# EUROPEAN PATENT SPECIFICATION

(11) **EP 1 254 651 B1**
(45) Date of publication and mention of the grant of the patent: **12.12.2007**
(21) Application number: 02290991.5
(22) Date of filing: 19.04.2002
(51) Int. Cl.: A61K 8/02, A61K 8/68, A61K 8/63, A61Q 13/00, C11D 3/50, C11D 3/32, C11D 7/32

(54) **Use of fragrance retaining composition**
Verwendung Duftstoff zurückhaltender Zusammensetzungen
Méthode de rétention de perfum

(30) Priority: 20.04.2001 JP 2001123394
(43) Date of publication of application: 06.11.2002
(73) Proprietor: Takasago International Corporation, Tokyo 144-8721 (JP)
(72) Inventor: Sakurai, Kazutoshi, Hiratsuka-shi, Kanagawa-ken (JP); Kawada, Izumi, Hiratsuka-shi, Kanagawa-ken (JP)
(74) Representative: Uchida, Kenji

(56) References cited:
- EP-A- 0 875 232
- EP-A- 0 920 852
- EP-A- 1 036 556
- EP-A- 1 166 769
- US-A- 5 344 650
- US-A- 5 688 752
- US-A- 5 776 480
- PATENT ABSTRACTS OF JAPAN vol. 2000, no. 20, 10 July 2001 (2001-07-10) & JP 2001 058939 A (MIKIMOTO PHARMACEUT CO LTD), 6 March 2001 (2001-03-06)

## Description

The present invention relates to a fragrance and cosmetic composition having a reinforced capacity for retaining the fragrances or perfumes used in a variety of cosmetics and fragrance products. The invention also concerns a fragrance and cosmetic product containing such a composition.

Fragrances and cosmetics are commonly used in: hair care products such as shampoo, rinse, conditioners and the like; body cleaners such as shower gel (or body shampoo) or liquid soap, cleansers for face washing and the like; skin care products such as creams, milky lotions, beauty washes and the like; fabric care products such as clothing detergents, clothing softeners and the like. When fragrances are added to such products, a proper measure is normally taken to retain these fragrances after their use. Such a measure includes, for instance:
(a) using an enduring fragrance or a retaining agent (solvents such as Hercolyn, alkylene glycol, alkyl citrate, benzyl benzoate, and fragrances such as Peru balsam, vetiver benzoin, labdanum, oak moss, patchouli), described in "Known and Conventional Technologies (Toiletry and Similar Products), p.182, 1984;
(b) increasing the amount of fragrances and using a fragrance preparation preferably containing a less volatile fragrance substance having a higher molecular weight; and
(c) including fragrances in a clathrate compound such as cyclodextrin.

However, in measure (a), even if the enduring fragrances or retaining agents are used, the fragrances are dissolved into water by action of surfactants and washed away. Desired fragrances can thus be hardly retained in hairs, on washed articles or on human skin. In measure (b), the fragrances contained in toilet articles or cosmetics become concentrated and intensified during use. Accordingly, the fragrances may be felt far different or removed from natural context. Moreover, such a measure is very costly. In method (c), the fragrances lose their essence during use.

Besides the above generally known technologies, there have been reported other specific methods, such as:
(d) method described in JP-A-HEI-5-279 237: a fragrance composition is supplemented with a humectant, oily substance and alcohol so as to improve the scent endurance;
(e) method described in JP-A-HEI-10-45 553: a detergent composition is supplemented with a specific amido-carboxylic acid as scent-retaining agent;
(f) method described in JP-A-2000-95 658: a fragrance substance having a specific chemical structure and vapour pressure is combined with an aromatic sulfonic acid or its salts e.g. 2-naphthalene sulfonic acid;
(g) method described in EP-A-679 715: a fragrance is supplemented with lard or a surfactant so as to improve the scent retention; and
(h) method described in JP-A-2000-96 079: the endurance of a fragrance or perfume adhered is improved by using tetra(2-hydroxypropyl) ethylenediamine.

However, in method (d), use is limited to alcohol products such as eau de cologne. A product type such as used with washing water is not suitable for this method, since the product cannot retain a sufficient amount of scent. Further, as triglycerides are added in large amount to such a product, it becomes sticky.

In method (e), amido-carboxylic acid has to be added in an amount equal to, or above, the amount of fragrance or perfume used. This creates economical handicap.

In method (f), the added sulfonic acid, when placed in acidic condition, may generate a typical sulfide odour. Further, the amount of sulfonic acid to be added is equal to, or above, that of fragrance or perfume used. This renders the method more expensive.

In method (g), the product tends to become viscous and to form a lard odour. Further, the lard and surfactant have to be added in an amount two or three times that of fragrance or perfume used. This renders the method economically less attractive.

In method (h), the endurance-imparting compound has also to be added in an amount equal to or higher than that of fragrance or perfume used. This renders the method more expensive.

In all the above methods, a large amount of effective compounds, relative to the quantity of fragrances, are required in order to retain efficiently the fragrances in hairs or on washed articles and human skin.

As can be observed from the foregoing, when fragrance products are commercially prepared, a certain amount of adjuvant compounds are added to the products. However, when the amount of adjuvant is small, no measure or method disclosed up to now can efficiently improve the fragrance effects, to such a level that scents are retained even after water washing or an unpleasant remnant odour is masked from washed material

The present invention intends to satisfy a long-dated desire to keep scents for a long time, and provide a fragrance composition with scent-retaining effect. The composition of the invention shows a marked effect in a small quantity, relative to the amount of fragrances used, on retaining the scents after washing, and on masking unpleasant odours of washed material. The invention also provides a cosmetics or fragrance product containing such a fragrance composition.

To this end, according to the invention, lipid compositions were searched which contain a ceramide and/or pseudo-ceramide, and it was found that, when a slight quantity of ceramide and/or pseudo-ceramide is/are added to a fragrance element, the fragrance endurance in cosmetics, hair care products, body-washer, fabric care products or the like was remarkably improved. It was further found that such a lipid composition greatly improved the endurance of the fragrances used in hairs after washing, on washed articles, or on human skin. Respective compositions are known from EP-A-0 875 232.

The invention thus relates to the use of a composition comprising a sphingolipid-based composition (A), and an ingredient (B) comprising at least one compound chosen from the group consisting of sterol-based compounds, for reinforcing the endurance of scents in a fragrance compound or composition.

Preferably, the sphingolipid-based composition (A) comprises:
a) at least a first ceramide ingredient (A₁) selected from the group consisting of 2-acylaminoalkan-1,3-diol and optically active forms thereof represented by the formula I: 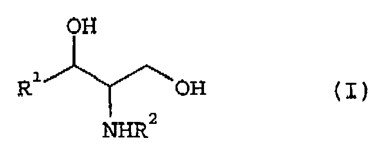 in which R¹ signifies a straight-chain alkyl group having 9 to 17 carbon atoms, and R² signifies a straight-chain acyl group having 14 to 24 carbon atoms; and
b) at least a second ceramide ingredient (A₂) selected from the group consisting of 2-acylaminoalkan-1,3-diol and optically active forms thereof represented by the formula II: 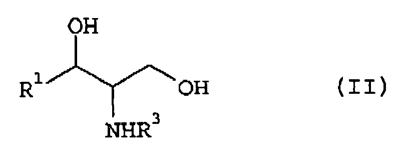
in which R¹ signifies a straight-chain alkyl group having 9 to 17 carbon atoms, and R³ signifies an acetyl group or a straight-chain acyl group having 2 to 24 carbon atoms with a hydroxy group at α- or β-position; and

Preferably yet, the fragrance-retaining composition further comprises at least one ingredient (C) selected from the group consisting of cholesterol esters and higher fatty acids.

Suitably, the first ceramide ingredient (A₁) comprises a (2S,3R)-2-acylaminoalkan-1,3-diol represented by the formula (IV): 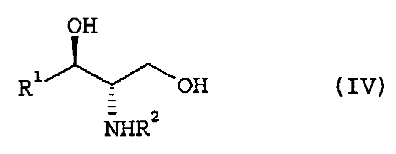 in which R¹ and R² are as defined for the formula (I).

Suitably yet, the second ceramide ingredient (A₂) comprises a (2S,3R)-2-acylaminoalkan-1,3-diol represented by the formula (V): 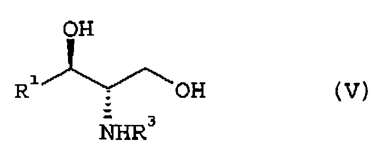 in which R¹ and R³ are as defined for the formula (II).

Alternatively, the sphingolipid-based composition (A) may comprise:
a) at least one pseudo-ceramide ingredient (A₃) selected from the group consisting of amide derivatives represented by the formula III: 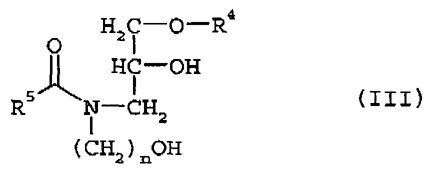 in which R⁴ signifies a straight-chain or branched, saturated or non-saturated hydrocarbon group having 10 to 26 carbon atoms; R⁵ signifies a straight-chain or branched, saturated or non-saturated hydrocarbon group having 9 to 25 carbon atoms; and n signifies a natural number ranging from 2 to 6; and
b) at least one ingredient (C) selected from the group consisting of cholesterol esters and higher fatty acids.

Preferably, the fragrance-retaining composition has a liquid-crystal structure.

The invention relates to a sphingolipid-related composition A which contains ceramide ingredients A₁ and A₂ and their optically active forms, i.e. 2-acylaminoalkan-1,3-diols, represented by the general formulae (I) and (II).

In the general formula (I), R¹ signifies a straight-chain alkyl group having 9 to 17 carbon atoms, and R² signifies a straight-chain acyl group having 14 to 24 carbon atoms.

Examples of R¹ include nonyl, decanyl, undecanyl, dodecanyl, tridecanyl, tetradecanyl, pentadecanyl, hexadecanyl and heptadecanyl groups.

Examples of R² include tetradecanoyl, pentadecanoyl, hexadecanoyl, heptadecanoyl, octadecanoyl, nonadecanoyl, eicosanoyl, heneicosanoyl, docosanoyl, tricosanoyl and tetracosanoyl groups.

In the ceramide ingredient A₁ of the formula (I), i.e. 2-acylaminoalkan-1,3-diols, examples in which R¹ has 15 carbon atoms include:
2-tetradecanoylaminooctadecan-1,3-diol;
2-pentadecanoylaminooctadecan-1,3-diol;
2-hexadecanoylaminooctadecan-1,3-diol;
2-heptadecanoylaminooctadecan-1,3-diol;
2-octadecanoylaminooctadecan-1,3-diol;
2-nonadecanoylaminooctadecan-1,3-diol;
2-eicosanoylaminooctadecan-1,3-diol;
2-heneicosanoylaminooctadecan-1,3-diol;
2-docosanoylaminooctadecan-1,3-diol;
2-tricosanoylaminooctadecan-1,3-diol; and
2-tetracosanoylaminooctadecan-1,3-diol.

However, the ceramide ingredients A₁ in which R¹ contains 15 carbon atoms are not limited to the above examples.

The ceramide compounds of the formula (I), in which R¹ has a number of carbon atoms different from 15, may be exemplified in the same manner as in the above case.

2-Acylaminoalkan-1,3-diols of the formula (I) according to the invention may be prepared through chemical synthesis. The compounds for the purpose of the invention may have racemic forms, a natural-type optically active form, a non-natural-type optically active form, or may be a mixture thereof.

In the formula (II) in which R¹ is defined as for the formula (I), R³ signifies acetyl group, or a straight-chain acyl group having 2 to 24 carbon atoms with a hydroxide group located in the α- or β-position. In the formula (II), examples of R¹ may be the same as for the formula (I).

Examples of R³ include acetyl, glycoloyl and lactoyl group, and a straight-chain acyl group having a hydroxyl group in the α-position, such as 2-hydroxybutanoyl, 2-hydroxypentanoyl, 2-hydroxyhexanoyl, 2-hydroxyheptanoyl, 2-hydroxyoctanoyl, 2-hydroxynonanoyl, 2-hydroxydecanoyl, 2-hydroxyundecanoyl, 2-hydroxydodecanoyl, 2-hydroxytridecanoyl, 2-hydroxytetradecanoyl, 2-hydroxypentadecanoyl, 2-hydroxyhexadecanoyl, 2-hydroxyheptadecanoyl, 2-hydroxyoctadecanoyl, 2-hydroxynonadecanoyl, 2-hydroxyeicosanoyl, 2-hydroxyheneicosanoyl, 2-hydroxydocosanoyl, 2-hydroxytricosanoyl and 2-hydroxytetracosanoyl; and a straight-chain acyl group having 3 to 24 carbon atoms with a hydroxyl group in the β-position, such as 3-hydroxypropanonyl, 3-hydroxybutanoyl, 3-hydroxypentanoyl, 3-hydroxyhexanoyl, 3-hydroxyheptanoyl, 3-hydroxyoctanoyl, 3-hydroxynonanoyl, 3-hydroxydecanoyl, 3-hydroxyundecanoyl, 3-hydroxydodecanoyl, 3-hydroxytridecanoyl, 3-hydroxytetradecanoyl, 3-hydroxypentadecanoyl, 3-hydroxyhexadecanoyl, 3-hydroxyheptadecanoyl, 3-hydroxyoctadecanoyl, 3-hydroxynonadecanoyl, 3-hydroxyeicosanoyl, 3-hydroxyheneicosanoyl, 3-hydroxydocosanoyl, 3-hydroxytricosanoyl and 3-hydroxytetracosanoyl.

In the ceramide compound A₂ of the formula (II), i.e. 2-acylaminoalkan-1,3-diols, examples in which R¹ contains 15 carbon atoms include:
2-acetylaminooctadecan-1,3-diol;
2-glycoloylaminooctadecan-1,3-diol;
2-lactoylaminooctadecan-1,3-diol;
2-(2'-hydroxybutanoyl)aminooctadecan-1,3-diol;
2-(2'-hydroxypentanoyl)aminooctadecan-1,3-diol;
2-(2'-hydroxyhexanoyl)aminooctadecan-1,3-diol;
2-(2'-hydroxyheptanoyl)aminooctadecan-1,3-diol;
2-(2'-hydroxyoctanoyl)aminooctadecan-1,3-diol;
2-(2'-hydroxynonanoyl)aminooctadecan-1,3-diol;
2-(2'-hydroxydecanoyl)aminooctadecan-1,3-diol;
2-(2'-hydroxytridecanoyl)aminooctadecan-1,3-diol;
2-(2'-hydroxytetradecanoyl)aminooctadecan-1,3-diol;
2-(2'-hydroxypentadecanoyl)aminooctadecan-1,3-diol;
2-(2'-hydroxyhexadecanoyl)aminooctadecan-1,3-diol;
2-(2'-hydroxyheptadecanoyl)aminooctadecan-1,3-diol;
2-(2'-hydroxyoctadecanoyl)aminooctadecan-1,3-diol;
2-(2'-hydroxynonadecanoyl)aminooctadecan-1,3-diol;
2-(2'-hydroxyeicosanoyl)aminooctadecan-1,3-diol;
2-(2'-hydroxyheneicosanoyl)aminooctadecan-1,3-diol;
2-(2'-hydroxydocosanoyl)aminooctadecan-1,3-diol;
2-(2'-hydroxytricosanoyl)aminooctadecan-1,3-diol;
2-(2'-hydroxytetracosanoyl)aminooctadecan-1,3-diol;
2-(3'-hydroxypropanoyl)aminooctadecan-1,3-diol;
2-(3'-hydroxybutanoyl)aminooctadecan-1,3-diol;
2-(3'-hydroxypentanoyl)aminooctadecan-1,3-diol;
2-(3'-hydroxyhexanoyl)aminooctadecan-1,3-diol;
2-(3'-hydroxyheptanoyl)aminooctadecan-1,3-diol;
2-(3'-hydroxyoctanoyl)aminooctadecan-1,3-diol;
2-(3'-hydroxynonanoyl)aminooctadecan-1,3-diol;
2-(3'-hydroxydecanoyl)aminooctadecan-1,3-diol;
2-(3'-hydroxyundecanoyl)aminooctadecan-1,3-diol;
2-(3'-hydroxydodecanoyl)aminooctadecan-1,3-diol;
2-(3'-hydroxytridecanoyl)aminooctadecan-1,3-diol;
2-(3'-hydroxytetradecanoyl)aminooctadecan-1,3-diol;
2-(3'-hydroxypentadecanoyl)aminooctadecan-1,3-diol;
2-(3'-hydroxyhexadecanoyl)aminooctadecan-1,3-diol;
2-(3'-hydroxyheptadecanoyl)aminooctadecan-1,3-diol;
2-(3'-hydroxyoctadecanoyl)aminooctadecan-1,3-diol;
2-(3'-hydroxynonadecanoyl)aminooctadecan-1,3-diol;
2-(3'-hydroxyeicosanoyl)aminooctadecan-1,3-diol;
2-(3'-hydroxyheneicosanoyl)aminooctadecan-1,3-diol;
2-(3'-hydroxydocosanoyl)aminooctadecan-1,3-diol;
2-(3'-hydroxytricosanoyl)aminooctadecan-1,3-diol;and
2-(3'-hydroxytetracosanoyl)aminooctadecan-1,3-diol.

However, the ceramide compounds A₂, in which R¹ has 15 carbon atoms, are not limited to the above examples.

The ceramide compounds of the formula (II), in which R¹ has a number of carbon atoms different from 15, may be exemplified in the same manner as in the above examples.

2-Acylaminoalkan-1,3-diols of the formula (II) according to the invention may be prepared through chemical synthesis. The compounds for the purpose of the invention may have racemic forms, a natural-type optically active form, a non-natural-type optically active form, or may be a mixture thereof.

The ceramide ingredients A₁ and A₂ of the invention include respectively an optically active (2S,3R)-2-acylaminoalkan-1,3-diol, i.e. having a (2S,3R)-configuration, which can be produced by known methods disclosed e.g. by JP-A-HEI 9-235 259 and JP-A-HEI 10-218 851.

For instance, (2S,3R)-2-acylaminooctadecan-1,3-diol is an optically active form of the ingredient A₁ or A₂ when R¹ has 15 carbon atoms, and is represented in the general formula (IV) or (V). This compound may be obtained by acylating, with a suitable acylating agent, (2S,3R)-2-aminooctadecan-1,3-diol which can in turn be obtained according to a method disclosed by JP-A-HEI 6-80 617 or JP-A-HEI 9-176 097.

The racemic form of 2-acylaminooctadecan-1,3-diol may be obtained by acylating, in the same manner, 2-aminooctadecan-1,3-diol which can be synthesised according to the method of D. SHAPIRO et al, disclosed in J. Am. Chem. Soc., 80, 2170, 1958.

The optically active form of ceramide ingredients A₁ or A₂ corresponds to (2S,3R)-2-acylaminoalkan-1,3-diol represented respectively by the general formula (IV) or (V).

In the general formula (IV), R¹ signifies a straight-chain alkyl group having 9 to 17 carbon atoms; and R² signifies a straight-chain acyl group having 14 to 24 carbon atoms.

Examples of R¹ and R² include the groups exemplified supra. In (2S,3R)-2-acylaminoalkan-1,3-diol, i.e. optically active form of ceramide ingredient A₁ of the general formula (IV), R¹ and R² are typically a straight-chain alkyl group having 15 carbon atoms and a straight-chain acyl group having 18 carbon atoms, respectively.

In the above (2S,3R)-2-acylaminoalkan-1,3-diol, examples where R¹ has 15 carbon atoms include:
(2S,3R)-2-tetradecanoylaminooctadecan-1,3-diol;
(2S,3R)-2-pentadecanoylaminooctadecan-1,3-diol;
(2S,3R)-2-hexadecanoylaminooctadecan-1,3-diol;
(2S,3R)-2-heptadecanoylaminooctadecan-1,3-diol;
(2S,3R)-2-octadecanoylaminooctadecan-1,3-diol;
(2S,3R)-2-nonadecanoylaminooctadecan-1,3-diol;
(2S,3R)-2-eicosanoylaminooctadecan-1,3-diol;
(2S,3R)-2-heneicosanoylaminooctadecan-1,3-diol;
(2S,3R)-2-docosanoylaminooctadecan-1,3-diol;
(2S,3R)-2-tricosanoylaminooctadecan-1,3-diol;
(2S,3R)-2-tetracosanoylaminooctadecan-1,3-diol;

However, the compounds are not limited to the examples cited above.

Examples of the general formula (IV) where R¹ has a number of carbon atoms other than 15 may be cited in the same manner as above.

In the general formula (V), R¹ signifies a straight-chain alkyl group having 9 to 17 carbon atoms; and R³ signifies acetyl group, or a straight-chain acyl group having 2 to 24 carbon atoms with a hydroxyl group in the α- or β-position.

In the formula (V), examples of R¹ may be the same as cited above.

Examples of R³ may include the groups described supra. In (2S,3R)-2-acylaminoalkan-1,3-diol, i.e. optically active form of ceramide A₂ of the general formula (V), R¹ is typically a straight-chain alkyl group having 15 carbon atoms, whilst R³ is typically an acetyl group or a straight-chain acyl group having 18 carbon atoms.

In the above (2S,3R)-2-acylaminoalkan-1,3-diol represented by the formula (V) of ingredients A₂, examples where R¹ has 15 carbon atoms include:
(2S,3R)-2-acetylaminooctadecan-1,3-diol;
(2S,3R)-2-glycoloylaminooctadecan-1,3-diol;
(2S,3R)-2-lactoylaminooctadecan-1,3-diol;
(2S,3R)-2-(2'-hydroxybutanoyl)aminooctadecan-1,3-diol;
(2S,3R)-2-(2'-hydroxypentanoyl)aminooctadecan-1,3-diol;
(2S,3R)-2-(2'-hydroxyhexanoyl)aminooctadecan-1,3-diol;
(2S,3R)-2-(2'-hydroxyheptanoyl)aminooctadecan-1,3-diol;
(2S,3R)-2-(2'-hydroxyoctanoyl)aminooctadecan-1,3-diol;
(2S,3R)-2-(2'-hydroxynonanoyl)aminooctadecan-1,3-diol;
(2S,3R)-2-(2'-hydroxydecanoyl)aminooctadecan-1,3-diol;
(2S,3R)-2-(2'-hydroxyundecanoyl)aminooctadecan-1,3-diol;
(2S,3R)-2-(2'-hydroxydodecanoyl)aminooctadecan-1,3-diol;
(2S,3R)-2-(2'-hydroxytridecanoyl)aminooctadecan-1,3-diol;
(2S,3R)-2-(2'-hydroxypentadecanoyl)aminooctadecan-1,3-diol;
(2S,3R)-2-(2'-hydroxyhexadecanoyl)aminooctadecan-1,3-diol;
(2S,3R)-2-(2'-hydroxyheptadecanoyl)aminooctadecan-1,3-diol;
(2S,3R)-2-(2'-hydroxyoctadecanoyl)aminooctadecan-1,3-diol;
(2S,3R)-2-(2'-hydroxynonadecanoyl)aminooctadecan-1,3-diol;
(2S,3R)-2-(2'-hydroxyeicosanoyl)aminooctadecan-1,3-diol;
(2S,3R)-2-(2'-hydroxyheneicosanoyl)aminooctadecan-1,3-diol;
(2S,3R)-2-(2'-hydroxydocosanoyl)aminooctadecan-1,3-diol;
(2S,3R)-2-(2'-hydroxytricosanoyl)aminooctadecan-1,3-diol;
(2S,3R)-2-(2'-hydroxytetracosanoyl)aminooctadecan-1,3-diol;
(2S,3R)-2-(3'-hydroxypropanoyl)aminooctadecan-1,3-diol;
(2S,3R)-2-(3'-hydroxybutanoyl)aminooctadecan-1,3-diol;
(2S,3R)-2-(3'-hydroxypentanoyl)aminooctadecan-1,3-diol;
(2S,3R)-2-(3'-hydroxyhexanoyl)aminooctadecan-1,3-diol;
(2S,3R)-2-(3'-hydroxyheptanoyl)aminooctadecan-1,3-diol;
(2S,3R)-2-(3'-hydroxyoctanoyl)aminooctadecan-1,3-diol;
(2S,3R)-2-(3'-hydroxynonanoyl)aminooctadecan-1,3-diol;
(2S,3R)-2-(3'-hydroxydecanoyl)aminooctadecan-1,3-diol;
(2S,3R)-2-(3'-hydroxyundecanoyl)aminooctadecan-1,3-diol;
(2S,3R)-2-(3'-hydroxydodecanoyl)aminooctadecan-1,3-diol;
(2S,3R)-2-(3'-hydroxytridecanoyl)aminooctadecan-1,3-diol;
(2S,3R)-2-(3'-hydroxytetradecanoyl)aminooctadecan-1,3-diol;
(2S,3R)-2-(3'-hydroxypentadecanoyl)aminooctadecan-1,3-diol;
(2S,3R)-2-(3'-hydroxyhexadecanoyl)aminooctadecan-1,3-diol;
(2S,3R)-2-(3'-hydroxyheptadecanoyl)aminooctadecan-1,3-diol;
(2S,3R)-2-(3'-hydroxyoctadecanoyl)aminooctadecan-1,3-diol;
(2S,3R)-2-(3'-hydroxynonadecanoyl)aminooctadecan-1,3-diol;
(2S,3R)-2-(3'-hydroxyeicosanoyl)aminooctadecan-1,3-diol;
(2S,3R)-2-(3'-hydroxyheneicosanoyl)aminooctadecan-1,3-diol;
(2S,3R)-2-(3'-hydroxydocosanoyl)aminooctadecan-1,3-diol;
(2S,3R)-2-(3'-hydroxytricosanoyl)aminooctadecan-1,3-diol;
(2S,3R)-2-(3'-hydroxytetracosanoyl)aminooctadecan-1,3-diol;
However, the compounds are not limited to the examples cited above.

Examples of the general formula (V) where R¹ has a number of carbon atoms different from 15 may be cited in the same manner as above.

The pseudo-ceramide compounds A₃ in the sphingolipid-based composition A according to the invention are amide derivatives represented by the formula (III).

In this formula, R⁴ signifies a straight- or branched-chain, saturated or non-saturated hydrocarbon group having 10 to 26 carbon atoms; R⁵ signifies a straight- or branched-chain, saturated or non-saturated hydrocarbon group having 9 to 25 carbon atoms; and n is an integer ranging from 2 to 6.

Examples of R⁴ include a decyl, undecyl, dodecyl, tridecyl, tetradecyl, pentadecyl, hexadecyl, heptadecyl, octadecyl, nonadecyl, eicosyl, heneicosyl, docosyl, tricosyl, tetracosyl, pentacosyl, or hexacosyl group and the like.

Examples of R⁵ include a nonyl, decyl, undecyl, dodecyl, tridecyl, tetradecyl, pentadecyl, hexadecyl, heptadecyl, octadecyl, nonadecyl, eicosyl, heneicosyl, docosyl, tricosyl, tetracosyl, or pentacosyl group and the like.

Among the amide derivatives represented by the formula (III), i.e. pseudo-ceramide compounds A₃, those in which R⁵ signifies a straight-chain saturated hydrocarbon group having 15 carbon atoms and n is 2, include:
N-(2-hydroxy-3-decyloxypropyl)-N-2-hydroxyethylhexadecanamide;
N-(2-hydroxy-3-undecyloxypropyl)-N-2-hydroxyethylhexadecanamide;
N-(2-hydroxy-3-dodecyloxypropyl)-N-2-hydroxyethylhexadecanamide;
N-(2-hydroxy-3-tridecyloxypropyl)-N-2-hydroxyethylhexadecanamide;
N-(2-hydroxy-3-tetradecyloxypropyl)-N-2-hydroxyethylhexadecanamide;
N-(2-hydroxy-3-pentadecyloxypropyl)-N-2-hydroxyethylhexadecanamide;
N-(2-hydroxy-3-hexadecyloxypropyl)-N-2-hydroxyethylhexadecanamide;
N-(2-hydroxy-3-heptadecyloxypropyl)-N-2-hydroxyethylhexadecanamide;
N-(2-hydroxy-3-octadecyloxypropyl)-N-2-hydroxyethylhexadecanamide;
N-(2-hydroxy-3-nonadecyloxypropyl)-N-2-hydroxyethylhexadecanamide;
N-(2-hydroxy-3-eicosyloxypropyl)-N-2-hydroxyethylhexadecanamide;
N-(2-hydroxy-3-heneicosyloxypropyl)-N-2-hydroxyethylhexadecanamide;
N-(2-hydroxy-3-docosyloxypropyl)-N-2-hydroxyethylhexadecanamide;
N-(2-hydroxy-3-tricosyloxypropyl)-N-2-hydroxyethylhexadecanamide;
N-(2-hydroxy-3-tetracosyloxypropyl)-N-2-hydroxyethylhexadecanamide;
N-(2-hydroxy-3-pentacosyloxypropyl)-N-2-hydroxyethylhexadecanamide;
N-(2-hydroxy-3-hexacosyloxypropyl)-N-2-hydroxyethylhexadecanamide.

Compounds of the formula (III) other than exemplified above may be cited in the same manner.

The amide derivatives of the formula (III), i.e. pseudo-ceramides of the compounds A-3 of the invention, may be prepared according to methods disclosed e.g. in JP-A-SHO-62-228 048, JP-A-SHO-63-216 812 and JP-A-SHO-64-4 236.

Sterol-based compounds as ingredient B of the invention include, for example, cholesterol, coprostanol, sitosterol, stigmasterol, ergosterol and the like. Amongst them, the most preferred sterol is cholesterol. The cholesterol may be of animal or plant origin, or may be a high-purity commercial product obtained by chemical syntheses (e.g. cholesterol produced by company WAKO JUNYAKU).

Cholesterol esters as ingredient C of the invention include, for example, esters such as cholesteryl oleate, cholesteryl stearate, cholesteryl hydroxystearate, and cholesteryl isostearate, and cholesterol hemiesters such as cholesteryl hemisuccinate, cholesteryl hemiglutarate and cholesteryl hemimaleate. These cholesterol esters may be chemically synthesised, or commercially available as high-purity products. Amongst them, cholesteryl hydroxystearate is preferably used, when a sphingolipid-based composition A is formed of ceramide ingredients A₁ and A₂. For instance, cholesteryl 12-hydroxystearate is manufactured by company NISSHIN SEIYU, and commercialised under the name "salacos HS". Conversely, cholesteryl isostearate is preferably used, when sphingolipid-based composition A is formed of pseudo-ceramide compounds A₃.

Examples of higher fatty acids as ingredient C include tetradecanoic acid (myristic acid), hexadecanoic acid (palmitic acid), octadecanoic acid (stearic acid), hydroxyoctadecanoic acid (hydroxystearic acid) and isostearic acid. In particular, stearic acid is preferably used. Those higher fatty acids may be commercially available compounds.

The lipid compositions used in the endurance-reinforced fragrance or cosmetic composition according to the invention may be prepared by blending a sphingolipid-based composition A, ingredient B, and optionally ingredient C, in an appropriate ratio.

When the sphingolipid-based composition A of the invention contains a two-compound system composed of a ceramide compound A₁ and a ceramide compound A₂, preferred is a lipid composition forming a liquid-crystal structure.

An appropriate ratio, in which a sphingolipid-based composition A (i.e. ceramide ingredients A₁ and A₂), sterol-based ingredient B, and optionally ingredient C, form a liquid-crystal structure, may change as a function of the type and purity of the ingredients used. However, this ratio can be easily determined by performing the tests using a specific type and ratio.

When the second ceramide ingredient A₂ are represented by the formula(e) (II) and/or (V), a lipid composition having a lamellar liquid-crystal structure may be prepared using 2-acetylaminoalkan-1,3-diol (i.e. R³ signifies acetyl group) according to a method disclosed in JP-A-HEI-11-12 118.

Likewise, when the ceramide compounds A₂ are represented by the formula(e) (II) and/or (V), a lipid composition having a lamellar liquid-crystal structure may be prepared using 2-acylaminoalkan-1,3-diol (where R³ signifies a straight-chain acyl group having 2 to 24 carbon atoms with a hydroxyl group in the α- or β-position) according to a method disclosed in Japan Patent Application No.2000-169 104, published on 18 December 2001 under No.2001-348 320. In this disclosure, the lipid composition is composed of; at least one compound (A₁) selected from the group consisting of 2-acylaminoalkan-1;3-diol of the formula (I) and optically active compounds thereof; at least one compound (A₂) selected from the group consisting of 2-acylaminoalkan-1;3-diol of the formula (II) and optically active compounds thereof, the acyl group having a hydroxyl group in the α- or β-position; and at least one compound (B) selected from the group consisting of sterols. Further, the ingredients A₁, A₂ and B are blended in an appropriate ratio, such that they form a liquid-crystal structure.

When the sphingolipid-based composition A used in the invention is a pseudo-ceramide ingredient A₃, the lipid composition preferably has an α-gel structure. Such a lipid composition has a lamellar liquid-crystal structure when prepared, but its structure is unstable and subsequently forms an α-gel structure.

An appropriate ratio between pseudo-ceramide ingredient A₃, ingredient B and ingredient C may depend on the type and purity of the ingredients used. However, this ratio can be easily determined by performing the tests using a specific type and ratio.

As to lipid compositions containing an amide derivative (pseudo-ceramide compound A₃ of the formula (III)) and having an α-gel structure, such lipid compositions may be prepared according to methods disclosed e.g. in JP-A-SHO-62-228 048, JP-A-SHO-63-216 812, JP-A-SHO-64-4 236 and J. Chem. Soc. Jap., 10, 1107-17, 1993.

In the endurance-reinforcing fragrance or cosmetic composition of the present invention, fragrances are supplemented with a small quantity of the above-mentioned lipid composition. As the result of this addition, the fragrance effect can be enhanced, and this effect remains even after water washing. The addition of the lipid composition can also mask unpleasant odour of washed articles. Besides, addition of such a lipid composition does not impede the balance of fragrances during use.

The amount of lipid composition to be supplemented in the composition of the invention may vary as a function of the type or combination of fragrances and lipid compositions used. However, in general, the lipid composition is added in an amount of 0.01 to 5.0% by weight, preferably 0.1 to 1.0 % by weight, relative to the amount of fragrances. For a final fragrance or cosmetic composition, the effect of lipid composition is not necessarily minimal when the quantity added is very small, nor necessarily improves when the quantity is multiplied. However, an excessive addition does not make sense economically. A suitable quantity to be added can be determined easily by making preparations with specific types and combinations of the lipid compositions.

As to fragrances for perfumed products, the lipid composition may be added in a proportion of 0.01 to 2.0 % by weight, preferably 0.1 to 1.0 % by weight, relative to the amount of fragrances.

As regards fragrances for hair care products e.g. shampoo, for rinse, and for conditioners, the lipid composition may be added, respectively, in a proportion of 0.1 to 1.0 % by weight, preferably 0.1 to 0.5 % by weight, relative to the amount of fragrances.

As to fragrances for body-washing e.g. shower gel or liquid soap, the lipid composition may be added in a proportion of 0.1 to 1.0 % by weight, preferably 0.1 to 0.2 % by weight, relative to the amount of fragrances.

As regards fragrances for face-washing cleansers, the lipid composition may be added in a proportion of 0.1 to 1.0 % by weight, preferably 0.1 to 0.5 % by weight, relative to the amount of fragrances.

As to fragrances for skin care products e.g. creams, for milky lotion, and for beauty wash, the lipid composition may be added, respectively, in a proportion of 0.1 to 1.0 % by weight, preferably 0.1 to 0.2 % by weight, relative to the amount of fragrances.

As to fragrances for fabric care products e.g. clothing detergents and for clothing softeners, the lipid composition may be added, respectively, in a proportion of 0.1 to 1.0 % by weight, preferably 0.1 to 0.2 % by weight, relative to the amount of fragrances.

There is no specific limit to the types of fragrance usable in the endurance-reinforced fragrance composition of the present invention. Both synthetic and natural fragrances may be used. These include a large spectrum of fragrance compounds described by ARCTANDER S., in an article entitled "Perfume and Flavour Chemicals", published in Montclair, N.J., USA, in 1969.

Examples of typical natural fragrance include: natural essential oil such as anis seeds, Ylang-Ylang, elemi, orris, orange, galbanum, clary sage, clove, coriander, sandalwood, citronella, cinnamon, jasmine, spearmint, cedar woods, geranium, celery, tangerine, Tonka beans, neroli, violet, patchouli, peach, vetiver, petitgrain, peppermint, Peru balsam, bergamot, eucalyptus, lilac, raspberry, lavender, lily-of-the-valley, lemon, lemon grass, lime and rose; and animal-origin fragrances such as amber, castrium, civet and musk.

Examples of typical synthetic fragrance include:
- hydrocarbons such as pinene, limonene, caryophyllene, longifolene and myrcene;
- alcohols such as cis-3-hexenol, Levosandol (manufactured by TAKASAGO INTERNATIONAL Corp.), p-t-butyrocyclohexanol, citronellol, geraniol, nerol, linalol, dihydrolinalol, tetrahydrolinalol, dihydromyrcenol, tetrahydromyrcenol, menthol, terpineol, borneol, isoborneol, isocamphylcyclohexanol, farnesol, cedrol, benzylalcohol, α-phenylethylalcohol, β-phenylethylalcohol, phenoxyethylalcohol, cinnamic alcohol, amylcinnamic alcohol, thymol and eugenol;
- ethers such as cineol, estragol, β-naphtholmethyether, β-naphtholethyether, diphenyloxide, cedrolmethyether, isoamylphenylethylether, Ambroxan (manufactured by HENKEL KgaA), Grisalva (manufactured by IFF), rose oxide, dihydrorose oxide, limonene oxide, menthofuran, Amber Core (manufactured by KAO Corp.);
- aldehydes such as fatty aldehydes having 9 to 12 carbon atoms, citronellal, citral, hydroxycitronellal, dimethyltetrahydrobenzaldehyde, myrac aldehyde (manufactured by IFF), Kovanol (manufactured by TAKASAGO INTERNATIONAL Corp.), Vernaldehyde (manufactured by GIVAUDAN ROURE S.A.), benzaldehyde, cyclamenaldehyde, Suzaral (manufactured by TAKASAGO INTERNATIONAL Corp.), Lilial (manufactured by GIVAUDAN ROURE S.A.), cinnamic aldehyde, hexylcinnamicaldehyde, vanillin, ethylvanillin, heliotropine and Heliobouquet (manufactured by TAKASAGO INTERNATIONAL Corp.);
- ketones such as cis-jasmone, dihydrojasmin, methyldihydrojasmonate ("Hedione" manufactured by FIRMENICH S.A.), cyclotene, damascenone, damascone, dynascone, ionone, methylionone, irone, Cashmeran (manufactured by IFF), Iso-E-Super (manufactured by IFF), carvon, menthone, acetylcedrene, isolongifolanone, raspberry ketone, acetophenone and benzophenone;
- esters such as γ-undecalactone, coumarin, linalyl formate, citronellyl formate, linalyl acetate, citronellyl acetate, geranyl acetate, terpinyl acetate, cedryl acetate, p-t-butylcyclohexyl acetate ("Vertenex" manufactured by IFF), 2-t-butylcyclohexyl acetate ("Verdox" manufactured by IFF), tricyclodecenyl acetate ("Erica acetate" manufactured by TAKASAGO INTERNATIONAL Corp.), benzyl acetate, phenylethyl acetate, styrallyl acetate, isoamyl acetate, rosephenone, dimethylbenzylcarbinyl acetate, Jasmal (manufactured by IFF), benzyl benzoate, benzyl salicylate, methyl atrarate, methyl anthranilate, dimethyl anthranilate, ethyl anthranilate, Auranthiol (manufactured by GIVAUDAN ROURE S.A.), ethyl trimethylcycohexanoate ("Thesarone" manufactured by TAKASAGO INTERNATIONAL Corp.) and Fruitate (manufactured by KAO Corp.); and
- musk-type compounds such as Muscone, Muscol, civetone, cyclopentadecanone, cyclohexadecanone, cyclohexadecenone ("Ambreton" manufactured by TAKASAGO INTERNATIONAL Corp.), Cyclopentadecanolide (manufactured by IFF), 10-oxahexadecanolide, ethylene brassylate ("Musk T" manufactured by TAKASAGO INTERNATIONAL Corp.), ethylenedodecanedioate, Celestolide (manufactured by IFF), Tonalide (manufactured by PFW), Galaxolide (manufactured by IFF), Traseolide (manufactured by QUEST INTERNATIONAL) and Phantolide (manufactured by PFW).

Each of the above fragrances may be used alone, or in a preparation containing at least two of them.

The above fragrances may be supplemented with one or several fragrance-retaining agent(s) commonly used in the prior art. Examples of such agent include propylene glycol, glycerine, hexylene glycol, dipropylene glycol, benzyl benzoate, triethyl citrate, diethyl phthalate and Hercolyn (methyl abietate).

The amount of enduring fragrance or cosmetic composition of the invention to be added to various cosmetic or fragrance products is not specifically limited, and may vary depending on the type of fragrances and cosmetics used and the fragrance products to which they are applied. This amount can be easily determined by making preparations with a specific type and combination. For a final cosmetics or fragrance product, the effect of fragrance or cosmetic composition is not necessarily minimal when the quantity added is very small, and not necessarily improves when the quantity is multiplied. However, an excessive addition is not economically desirable. A suitable quantity to be added can be determined easily by making preparations with specific types and combinations of the fragrance or cosmetic composition.

As to fragrances for perfumed products, the fragrance or cosmetic composition of the invention may be used in an approximate proportion of 15 to 40 % by weight relative to the amount of perfumed water, 10 to 20 % by weight relative to the amount of eau de parfum, 5 to 15 % by weight relative to the amount of eau de toilette, and 2 to 10 % by weight relative to the amount of eau de cologne.

As to hair care products, the fragrance or cosmetic composition of the invention may be used in an approximate proportion of 0.2 to 2.0 % by weight relative to the amount of shampoo, 0.3 to 1.0 % by weight relative to the amount of rinse, and 0.3 to 2.0 % by weight relative to the amount of conditioner.

As to body-cleansing products, the fragrance or cosmetic composition of the invention may be used in an approximate proportion of 0.3 to 3.0 % by weight relative to the amount of shower gel, and 0.1 to 2.0 % by weight relative to the amount of face-wash cleanser.

As to skin care products, the inventive fragrance or cosmetic composition may be used in an approximate proportion of 0.01 to 5.0 % by weight relative to the amount of cream, 0.01 to 4.0 % by weight relative to the amount of milky lotion, and 0.01 to 1.0 % by weight relative to the amount of beauty wash (toilet water).

As to fabric care products, the inventive fragrance or cosmetic composition may be used in an approximate proportion of 0.01 to 2.0 % by weight relative to the amount of clothing detergent, and 0.05 to 2.0 % by weight relative to the amount of clothing softener.

The exact constitution of the fragrance or cosmetic composition of the invention may depend on the contents of a cosmetic or fragrance product to be prepared. Examples of these contents are described herebelow.

A perfumed product generally contains a fragrance composition, an alcohol and distilled water. In this product, the range of fragrance composition to be added is varied as stated supra.

A shampoo typically contains, though not specifically limited, 5 to 25 % by weight of anionic surfactant (e.g. alkyl sulphate salts and salts of polyoxyethylenealkylether sulphate) and/or amphoteric surfactant (e.g. betaine); 0 to 10 % by weight of non-ionic surfactant and/or cationic surfactant; 0 to 5 % by weight of additives (e.g. a metal-ion enclosing agent, a pH-adjusting agent, an antiseptic, a cationic macromolecule, a humectant, a clouding agent, a colorant, a pigment, a brightener, a drug, an emulsifier, a hair-protecting agent, a viscosity-adjusting agent, an oxidation-preventing agent, a conditioner, a sun-screening agent, an antidandruff agent and a salt); and 50 to 95 % by weight of water.

A rinse typically contains, though not specifically limited, 5 to 30 % by weight of cationic surfactant and/or amphoteric surfactant; 0 to 20 % by weight of oily substance, e.g. a higher alcohol, a hydrocarbon oil, an ester oil and a silicone oil; 0 to 20 % by weight of humectant; 0 to 5 % by weight of additives, e.g. a pH-adjusting agent, an anti-septic, an oxidation-preventing agent, a colorant, a pigment, a brightener, a drug, an emulsifier, a hair-protecting agent, a viscosity-adjusting agent; and 30 to 95 % by weight of water.

A conditioner typically contains, though not specifically limited, 5 to 30 % by weight of cationic surfactant and/or amphoteric surfactant; 0 to 20 % by weight of oily substance, e.g. a higher alcohol, a hydrocarbon oil, an ester oil and a silicone oil; 0 to 20 % by weight of humectant; 0 to 5 % by weight of additives, e.g. a pH-adjusting agent, an anti-septic, an oxidation-preventing agent, a colorant, a pigment, a brightener, a drug, an emulsifier, a hair-protecting agent, a viscosity-adjusting agent; and 30 to 95 % by weight of water.

A shower gel typically contains, though not specifically limited, 5 to 75 % by weight of anionic surfactant and/or amphoteric surfactant; 5 to 15 % by weight of non-ionic surfactant; 0 to 5 % by weight of additives, e.g. a chelating agent, a skin conditioner, a skin softener (an emollient), an oxidation-preventing agent, an anti-septic, dyestuffs, a pigment and a salt; and 3 to 90 % by weight of water.

A face-wash cleanser typically contains, though not specifically limited, 5 to 75 % by weight of anionic surfactant and/or amphoteric surfactant; 5 to 15 % by weight of non-ionic surfactant; 0 to 5 % by weight of additives, e.g. a chelating agent, a skin conditioner, a skin softener (emollient), an oxidation-preventing agent, an anti-septic, dyestuffs, a pigment and a salt; and 3 to 90 % by weight of water.

A cream typically contains, though not specifically limited, 50 to 80 % by weight of oily substance; 5 to 15 % by weight of emulsifier; 0 to 5 % by weight of additives, e.g. a surfactant, a humectant, an oxidation-preventing agent, an anti-septic, dyestuffs, a pigment, a drug and a salt; and 30 to 90 % by weight of water or hydrophilic solvent e.g. ethanol and polyols.

A milky lotion typically contains, though not specifically limited, 5 to 15 % by weight of oily substance; 2 to 10 % by weight of emulsifier; 0 to 5 % by weight of additives, e.g. a surfactant, a hydrophilic macromolecule, a humectant, an oxidation-preventing agent, an anti-septic, dyestuffs, a pigment, a drug, a chelating agent, an ultraviolet-absorbent, a dispersant, a colour-fading preventing agent, a buffer agent and a salt; and 80 to 93 % by weight of water or hydrophilic solvent e.g. ethanol and polyols.

A beauty wash typically contains as main ingredients, though not specifically limited, a purified water, an alcohol (e.g. ethanol and isopropanol), a humectant (e.g. glycerine, propyleneglycol, dipropyleneglycol and polyethyleneglycol), a softener, an emollient (e.g. an ester oil and a plant oil), a solubilising agent (e.g. a surfactant having a high HLB), a buffer agent (e.g. citric acid, lactic acid, amino acids), a viscosity-thickener (e.g. alginic acid salt, a cellulose derivative, pectin, xanthan gum, carboxyvinyl polymer, an acrylic acid-type polymer), and an anti-septic (e.g. methylparaben and phenoxyethanol).

A clothing detergent (powder) typically contains, though not specifically limited, 5 to 30 % by weight of anionic surfactant and/or amphoteric surfactant; 1 to 10 % by weight of non-ionic surfactant; 0 to 5 % by weight of foaming agent e.g. alkanol amide; 35 to 60 % by weight of builder and/or fillers; 0 to 15 % by weight of bleacher and/or bleacher precursor; 0 to 15 % by weight of additives, e.g. a fluorescent whitener, a chelating agent, a re-sticking-preventing agent, an enzyme and dyestuffs; and 2 to 15 % by weight of water.

A clothing detergent (liquid) typically contains, though not specifically limited, 5 to 40 % by weight of anionic surfactant; 1 to 20 % by weight of non-ionic surfactant; 0 to 30 % by weight of builder and/or metallic ion-enclosing agent; 0 to 15 % by weight of alcohol and/or emulsifier; 1 to 5 % by weight of additives, e.g. a re-sticking-preventing agent, a fluorescent whitener, an enzyme, a textile conditioner and dyestuffs; and 40 to 60 % by weight of water.

A clothing softener typically contains, though not specifically limited, 4 to 50 % by weight of cationic surfactant; 0 to 5 % by weight of a lubricant e.g. lanolin and a fatty acid; 0.5 to 5 % by weight of additives, e.g. an anti-septic, dyestuffs, a pH-adjusting agent, a co-solvent, a clouding agent and electrolytes; and 49.5 to 95.5 % by weight of water.

The cosmetic or fragrance products containing the endurance-reinforced fragrance or cosmetic composition of the invention may further include one or several commonly used ingredient(s), such as powder ingredients, liquid oils-and-fats, solid oils-and-fats, wax, hydrocarbons, plant extracts, Chinese medicinal ingredients, higher alcohols, lower alcohols, esters, long-chain fatty acids, surfactants (non-ionic surfactants, anionic surfactants, cationic surfactants, amphoteric surfactabts), sterols, polyols, humectants, hydrophilic macromolecular compounds, viscosity-thickeners, coatings, bactericides, anti-septic agents, ultraviolet absorbents, retention agents, cool-feeling imparting agents, warm-feeling imparting agents, masking agents, whitening agents, metal ion-enclosing agents, sugar, amino acids, organic amines, synthetic resin emulsions, pH-adjusting agents, skin-nourishing agents, vitamins, oxidation-preventing agents, ceramides, natural moisturising factors (NMF), collagens, urea, oily substance, powder, Functional Beads, capsules, metal-chelating agents, inorganic salts and organic salts.

The enduring fragrance or cosmetic composition of the invention may be prepared, together with the above-mentioned ingredients, according to known methods. The nature of the composition is not specifically limited, and may be in the form of liquid, gel or aerosol paste.

The cosmetic or fragrance products containing the above enduring fragrance or cosmetic composition may be applied to hairs, head skin and skin, but not limited specifically thereto.

Examples of the perfumed products include perfumed water, eau de parfum, eau de toilette and eau de cologne.

Likewise, examples of the hair care products include shampoos, rinses and conditioners.

Further, examples of the body cleansers include shower gels and face-washing cleansers.

Typically, examples of the skin care products include creams, milky lotions and beauty wash (toilet water, lotion).

Further, examples of the fabric care products include clothing detergents and clothing softeners.

The above, and the other objects of the present invention will be made apparent from the following description of the preferred embodiments. However, the invention is not limited to the means, materials and embodiments disclosed below. In the following embodiments, the terms "%" and "parts" signify "% by weight" and "parts by weight" respectively, unless otherwise stated.

The devices used for analyses are:
- Gas chromatograph: HP-6890 (Agilent Technology);
- Column: DB-5MS, 0.25mm x 30m, 0.25µm (J&W Scientific);
- Injection temperature: 250°C;
- Column temperature: 300°C, increased from 50°C (after 5 min) at a rate of 5°C/min;
- Detection temperature: 250°C;
- Mass spectrum: HP-5973 Mass spectrometer (Agilent Technology).

### [Preparation Example 1]

### Preparation of lipid composition 1 having a liquid-crystal structure

The compounds used as ingredients A₁, A₂, B and C are, respectively, (2S,3R)-2-octadecanoylaminooctadecan-1,3-diol (i.e. R¹ = C₁₅H₃₁, R² = C₁₇H₃₅ in the formula IV), (2S,3R)-2-acetylaminooctadecan-1,3-diol (i.e. R¹ = C₁₅H₃₁, R³ = CH₃ in the formula V), cholesterol and cholesteryl 12-hydroxystearate. These compounds were mixed in a weight proportion of A₁:A₂:B:C = 2:1:1:2. The resultant mixture was dissolved in chloroform and homogenised. The mixture was heated to remove the chloroform solvent completely, and then allowed to stand in the air, to yield a mixture paste having a liquid-crystal structure.

### [Preparation Example 2]

### Preparation of lipid composition 2 having a liquid-crystal structure

The compounds used as ingredients A₁, A₂ and B are, respectively, (2S,3R)-2-octadecanoylaminooctadecan-1,3-diol (i.e. R¹ = C₁₅H₃₁, R² = C₁₇H₃₅ in the formula IV), (2S,3R)-2-(2'-hydroxyhexadecanoyl) aminooctadecan-1,3-diol (i.e. R¹ = C₁₅H₃₁, R³ = C₁₄H₂₉CH(OH) in the formula V), cholesterol. These compounds were mixed in a weight proportion of A₁:A₂:B = 2:2:3. The resultant mixture was dissolved in chloroform and homogenised. The mixture was heated to remove the chloroform solvent completely, and then allowed to stand in the air, to yield a mixture paste having a liquid-crystal structure.

### [Preparation Example 3]

### Preparation of lipid composition 3 having an α-gel structure

The compounds used as pseudo-ceramide ingredient A₃, sterol-based ingredient B, a first ingredient C (C₁) and a second ingredient C (C₂) are, respectively, N-(2-hydroxy-3-hexadecyloxypropyl)-N-2-hydroxyethyl hexadecanamide (i.e. R⁴ = C₁₆H₃₃, R⁵ = C₁₅H₃₁, n = 2 in the formula (III)), cholesterol, cholesteryl isostearate and stearic acid. These compounds were mixed in a weight proportion of A₃:B:C₁:C₂ = 10:3:1:6. The resultant mixture was dissolved in chloroform and homogenised. The mixture was heated to remove the chloroform solvent completely, and then allowed to stand in the air, to yield a mixture paste. The mixture paste has an unstable lamellar liquid-crystal structure at the outset, which later forms an α-gel structure.

### [First Embodiment, Comparative Example 1]

### Preparation of a fragrance composition

The lipid composition 1 obtained by Preparation Example 1 was added to geraniol (manufactured by TAKASAGO INTERNATIONAL Corp.) in a proportion of 0.1 %, and dissolved therein, to obtain Fragrance Composition 2 (1^{st} Embodiment), whereas Fragrance Composition 1 was prepared without adding the above lipid composition to geraniol (Comparative Example 1).

A paper was spotted with Fragrance Composition 1, then another paper with Fragrance Composition 2, respectively, in an amount of 0.10g. The perfumed papers were subjected to a group of 10 panellists, who evaluated the intensity of geraniol scent emanating from those papers.

### Results

All the ten panellists detected no geraniol scent, after 24 hours, from the paper spotted with Fragrance Composition 1. By contrast, they detected geraniol scents, even after 4 days, from the paper spotted with Fragrance Composition 2, thereby confirming a strong fragrance-retaining effect of the fragrance composition of the invention.

### [Second Embodiment, Comparative Example 2]

### Preparation of a fragrance composition

The lipid composition 1 obtained by Preparation Example 1 was added to a 30 % ethanol solution of ethylene brassylate ("Musk T" manufactured by TAKASAGO INTERNATIONAL Corp.) in a proportion of 0.1 %, and dissolved therein, to obtain Fragrance Composition 4 (2^{nd} Embodiment), whereas Fragrance Composition 3 was prepared without adding the above lipid composition to a 30 % ethanol solution of ethylene brassylate (Comparative Example 2).

A paper was spotted with Fragrance Composition 3, then another paper with Fragrance Composition 4, respectively, in an amount of 0.10g. The perfumed papers were subjected to a group of 10 panellists, who evaluated the intensity of ethylene brassilate scent emanating from those papers, respectively after 24 and 48 hours.

### Results

Table 1 shows the evaluation results after 24 and 48 hours, in which digits indicate the number of panellists, who compared Fragrance Composition 4 to Fragrance Composition 3 and expressed opinions listed in the first column.

**Table 1**

| Evaluation of Fragrance Composition 4 over Fragrance Composition 3 | After 24 hours | After 48 hours |
|---|---|---|
| Weak | 0 | 0 |
| Relatively weak | 0 | 0 |
| Same | 0 | 1 |
| Relatively strong | 6 | 7 |
| Strong | 4 | 2 |

As can be seen in Table 1, all the ten panellists felt, after 24 and 48 hours, a stronger musk scent in Fragrance Composition 4 than in Fragrance composition 3. This shows a significant fragrance-retaining effect of the lipid composition 1 of the invention.

### [Third Embodiment, Comparative Examples 3 to 5]

### Preparation of a fragrance composition

Fragrance Composition 5 (Table 2) was prepared with a rose-like fragrance, but without adding the lipid composition 1 of Preparation Example 1, to obtain Comparative Example 5. The above lipid composition was added to Fragrance Composition 5 in a proportion of 0.5 % and dissolved therein, to obtain Fragrance Composition 6 (3^{rd} Embodiment). Likewise, Fragrance Composition 7 was prepared by adding triolein (glyceride trioleate) to Fragrance Composition 5 in a proportion of 3 % and dissolved therein (Comparative Example 3); Fragrance Composition 8 was prepared by adding Hercolyn (methyl abietate) to Fragrance Composition 5 in a proportion of 3 % and dissolved therein (Comparative Example 4);.

Papers were spotted with Fragrance Compositions 5, 6, 7 and 8, respectively, in an amount of 0.10g. The perfumed papers were subjected to a group of 10 panellists, who evaluated, after 24 and 48 hours, the strength of rose-like scents emanating from those papers.

**Table 2**

| Preparation of a rose-like fragrance (Fragrance Composition 5) | |
|---|---|
| Geraniol | 400 parts |
| Geranium oil | 60 |
| Nerol | 200 |
| β-phenylethylalcohol | 90 |
| α-ionone | 40 |
| Eugenol | 10 |
| Linalool | 15 |
| Geranyl acetate | 35 |
| Iris oil | 10 |
| Rhodinyl formate | 20 |
| Nonanal | 1 |
| Undecanal | 2 |
| Dodecanal | 2 |
| Vanillin | 7 |
| Musk ketone | 8 |
| Rose oil | 100 |

The scent intensities of Fragrance Compositions 5, 6, 7 and 8 were compared after 24 and 48 hours. The results are presented in Table 3, in which Fragrance Compositions ("F.C.") are arranged from left-hand side to right-hand side in the decreasing order of scent intensity.

**Table 3**

| | |
|---|---|
| After 24h | F.C.6 > F.C.8 = F.C.7 > F.C.5 |
| After 48h | F.C.6 >> F.C.8 = F.C.7 > F.C.5 |

Table 3 shows that all the ten panellists perceived the strongest rose-like scent in Fragrance Composition 6 of the invention, respectively after 24 and 48 hours, suggesting a strong scent-retaining effect of the inventive lipid composition 1.

### [Fourth Embodiment, Comparative Example 6]

### Preparation of a shampoo

In Comparative Example 6, a base shampoo was prepared according to Formulation 1 in Table 4, and Fragrance Composition for Shampoos "A-1" (Table 5) was added to the base shampoo to obtain a product "Shampoo 1".

**Table 4**

| Formulation 1 for "Shampoo-1" | |
|---|---|
| Purified water | 42.13 % |
| Ortho-[2-hydroxy-3-(trimethylammonio)propyl] hydroxyethyl cellulose chloride | 0.6 |
| Polyoxyethylenelaurylether sodium sulphate (3 E.O.; 25 %) | 40.0 |
| Disodium mono-(lauroylethanolamide polyoxyethylene) sulfosuccinate (5 E.O.) | 5.00 |
| 2-alkyl-N-carboxymethyl-N-hydroxyethylimidazolium betaine | 5.00 |
| Palm oil fatty acid diethanolamide | 4.00 |
| Glycerine | 0.10 |
| Distearic acid ethyleneglycol | 1.50 |
| Citric acid | 0.22 |
| Potassium chloride | 0.30 |
| Methyl p-oxybenzoate | 0.20 |
| Propyl p-oxybenzoate | 0.10 |
| Ethyl p-oxybenzoate | 0.10 |
| Tetrasodium edetate | 0.05 |
| Fragrance Composition for Shampoos "A-1" | 0.50 |

**Table 5**

| Fragrance Composition for Shampoos "A-1" | |
|---|---|
| Lilial (GIVAUDAN ROURE) | 70 parts |
| Iso-E-super (IFF) | 20 |
| Hexyl salicylate | 25 |
| Methyl dihydrojasmonate | 150 |
| α-hexyl cinnamic aldehyde | 40 |
| Citronellol | 25 |
| Galaxolide (IFF) | 50 |
| Benzyl benzoate | 50 |
| Benzyl salicylate | 10 |
| Lemon oil | 70 |
| Musk T (TAKASAGO) | 30 |
| Methy ionone | 25 |
| Benzyl acetate | 30 |
| Tonalide (PFW) | 15 |
| Phenylethylalcohol | 20 |
| Hydroxycitronellal | 70 |
| Rose oil | 50 |
| Dipropylene glycol | 250 |

In 4^{th} Embodiment, the lipid composition 1 of Preparation Example 1 was added to Fragrance Composition "A-1" in a proportion of 0.2 % to obtain Fragrance Composition "A-2". The latter was added to the base shampoo to obtain a product "Shampoo-2".

A first bundle of homogenous human hairs (20 g) were dipped in 50 ml of warm water (40°C) for 20 min, and washed with 1 g of Shampoo-1. A second bundle of homogenous human hairs (20 g) were treated the same way and washed with Shampoo-2. Each bundle was rinsed with 100 ml of warm water, wiped with a towel, and dried.

A group of ten panellists evaluated scent intensities of those hairs in a wet state, and after 24 hours in a dried state.

### Evaluation rating

Panellists' evaluations on the intensity of remaining scent (enduring fragrance) were rated into 5 categories on the basis of the criteria defined herebelow:
Rating 5: strong
Rating 4: relatively strong
Rating 3: about the same
Rating 2: relatively weak
Rating 1: weak

### Results

As can be seen in Table 6, no significant difference between the shampoos used was detected immediately after the use. However, tests after one, two and 24 hours of use revealed that the hairs washed with Shampoo-2 (using the lipid composition of the invention) retained a remarkably strong remaining scent.

**Table 6**

| Evaluation of remaining scents | | | | |
|---|---|---|---|---|
| | Immediately after washing | After 1 h | After 2 h | After 24 h |
| Shampoo-1* (Com. Ex. 6) | 3.50 | 3.50 | 2.00 | 1.90 |
| Shampoo-2** (4^{th} Embodiment) | 3.50 | 3.40 | 3.10 | 2.30 |

| | | | | |
|---|---|---|---|---|
| *Shampoo-1 contains Fragrance Composition "A-1", but no lipid composition of the invention; **Shampoo-2 contains Fragrance Composition "A-2" (0.2 % of lipid composition of the invention). | | | | |

### Complementary remarks

The water phase collected after hair-washing was subjected to scent evaluation, and was found that the water phase of Shampoo-2 kept a much lesser scent intensity than that of Shampoo-1.

The above results indicate that Shampoo-2 containing the inventive lipid composition displays a marked scent-retaining capacity one, two and 24 hours after hair-washing, compared to Shampoo-1.

### [5^{th} to 8^{th} Embodiments, Comparative Example 7]

### Preparation of shampoo

The lipid composition of the Preparation Example 1 was added to Fragrance Composition for Shampoo "A-1", respectively in a proportion of 0.1 %, 0.25 %, 0.5 % and 1.0 %, to obtain, respectively, Fragrance Compositions "A-3", "A-4", "A-5" and "A-6".

Subsequently, a corresponding number of base shampoos of 4^{th} Embodiment were prepared according to Formulation 1 in Table 4, and Fragrance Compositions "A-1", "A-3", "A-4", "A-5" and "A-6" were added to the corresponding base shampoo, to obtain, respectively, Shampoo-1 (Comparative Example 7), Shampoo-3, Shampoo-4, Shampoo-5 and Shampoo-6 (5^{th} to 8^{th} embodiments).

Evaluation tests were carried out as mentioned in the 3^{rd} Embodiment. The results are presented in Table 7.

**Table 7**

| Evaluation of remaining scents | | | |
|---|---|---|---|
| | Immediately after washing | After 2 h | After 24 h |
| Shampoo-1 (Com. Ex. 7) | 3.50 | 2.00 | 1.90 |
| Shampoo-3 (5^{th} Embodiment) | 3.50 | 3.00 | 2.10 |
| Shampoo-4 (6^{th} Embodiment) | 3.50 | 3.10 | 2.30 |
| Shampoo-5 (7^{th} Embodiment) | 3.50 | 3.10 | 3.00 |
| Shampoo-6 (8^{th} Embodiment) | 3.50 | 3.40 | 3.00 |

As can be seen from above, Shampoo-3 to Shampoo-6 (i.e. 5^{th} to 8^{th} Embodiments in which the inventive lipid compositions are used), exhibit an improved scent-retaining capacity, compared to Shampoo-1 (i.e; Comparative Example 7 in which no lipid composition is used). Moreover, the intensity and endurance of fragrances are proportional to the quantity of lipid composition added.

In the 5^{th} to 8^{th} Embodiments and Comparative Example 7 above, hairs were dried for 24 hours and subjected to analyses by "Headspace GC-MS".

In particular, the intensity of hairs' smell was first evaluated. The hairs were then enclosed e.g. in a mayonnaise bottle having a volume of 300 ml, and their headspace was compared. To this end, a fibre assembly for Solid Phase Micro Extraction "SPME", manufactured by Supelco Inc. for gas chromatography use, was inserted into the bottle up to the level of the deposed hairs, and collected an air sample for 10 min. The air sample was then introduced into an apparatus "GC-MS" and analysed.

The area value of main fragrance ingredients, detected in the hairs after 24 hours of drying, was calculated, giving the following results.

### Total amounts of fragrance ingredients detected by "Headspace GC-MS"

| | |
|---|---|
| Comparative Example 7 | : 4, 342, 998 |
| 5^{th} Embodiment | : 6, 226, 553 |
| 6^{th} Embodiment | : 7, 221, 492 |
| 7^{th} Embodiment | : 9, 345, 258 |
| 8^{th} Embodiment | : 12, 841, 322 |

As is obvious from above, the hairs washed with the shampoos containing the inventive lipid compositions (5^{th} to 8^{th} Embodiments) contain a higher fragrance amount 24 hours after the use, detected by "Headspace GC-MS", as compared to Shampoo-1 (Comparative Example 7) containing no such lipid composition.

In the above analyses, all the main fragrance ingredients (benzyl acetate, citronellol, lilial, Iso-E-super, hexyl salicylate, hexyl cinnamic aldehyde and the like) were detected in a higher level in 5^{th} to 8^{th} Embodiments than in Comparative Example 7.

The endurance of the fragrances in the shampoos containing the inventive lipid composition can thus be confirmed by both smelling tests and the physical analyses effected by "Headspace GC-MS".

### [9^{th} Embodiment, Comparative Example 8]

### Evaluation in detergents (towels and socks)

A base powder detergent was prepared according to Formulation 2 in Table 8 to give Comparative Example 8. To this was added Fragrance Composition for Powder Detergents "B-1" (Table 9) to give a product "Powder Detergent-1".

As a ninth Embodiment, the lipid composition 1 of Preparation Example 1 was added to Fragrance Composition "B-1" according to Formulation 3 in Table 10 in a proportion of 0.15 %, to obtain Fragrance Composition "B-2". The latter was used to prepare a product "Powder Detergent-2".

**Table 8**

| Formulation 2 for Powder Detergent-1 | |
|---|---|
| Anionic surfactant (LAS): sodium alkylbenzenesulfonate | 20 parts |
| Soap | 1 |
| Zeolite | 12 |
| Polycarboxylate | 1 |
| Soda ash | 20 |
| Mirabilite | 30 |
| Fluorescent agent | 0.1 |
| B-1 | 0.5 |

**Table 9**

| Fragrance Composition for Powder Detergents "B-1" | |
|---|---|
| Ambroxan (HENKEL KgaA) | 15 parts |
| Benzyl acetate | 25 |
| Citronellol | 50 |
| Coumarin | 60 |
| Tricyclodecenyl acetate | 80 |
| Cyclamen aldehyde | 20 |
| Dihydromyrcenol | 80 |
| Diphenyl oxide | 20 |
| Geraniol | 50 |
| α-hexylcinnamic aldehyde | 155 |
| Orange terpene | 20 |
| Linallol | 50 |
| Nopyl acetate | 20 |
| Galaxolide (IFF) | 80 |
| Rose oxide | 5 |
| Tonalide (PFW) | 50 |
| 4-butylcyclohexyl acetate | 70 |
| 2-butylcyclohexyl acetate | 100 |

**Table 10**

| Formulation 3 for Powder Detergent-2 | |
|---|---|
| Anionic surfactant (LAS): sodium alkylbenzenesulfonate | 20 parts |
| Soap | 1 |
| Zeolite | 12 |
| Polycarboxylate | 1 |
| Soda ash | 20 |
| Mirabilite | 30 |
| Fluorescent agent | 0.1 |
| B-2 | 0.5 |

Cotton towels and cotton socks were washed with the powder detergents obtained above and rinsed, and the water was removed.

A group of ten panellists evaluated the intensity of scents emanating from the towels and socks immediately after the water-removal, and after 2 and 24 hours.

### Evaluation rating

Panellists' evaluations on the intensity of remaining scent (enduring fragrance) were rated into 5 categories on the basis of the definitions mentioned for 4^{th} Embodiment supra.

**Table 11**

| Evaluation of remaining scents | | | |
|---|---|---|---|
| | Immediately after washing | After 2 h | After 24 h |
| Powder Detergent-1 (Com. Ex. 8) | 2.90 | 2.80 | 1.70 |
| Powder Detergent-2 (9th Embodiment) | 3.50 | 3.20 | 2.40 |

As can be seen from the above table, Powder Detergent 2 (9^{th} Embodiment) containing the inventive lipid composition produce a markedly better scent retention effect, immediately after washing, and after 2 and 24 hours, compared to Powder Detergent-1 (Comparative Example-8) containing no such lipid composition.

### [Tenth Embodiment, Comparative Examples 9 to 13]

### Preparation of softeners

In 10^{th} Embodiment, the lipid composition 1 of Preparation Example 1 was added to Fragrance Composition for Softener "C-1" in a proportion of 0.2 %, to give Fragrance Composition "C-2".

A commercially available non-perfumed softener ("Downy care", manufactured by PROCTOR GAMBLE) was then supplemented with Fragrance Composition "C-2" in a proportion of 0.5 %, to obtain a product "Softener-1".

In Comparative Examples 10 to 13, Fragrance Composition "C-1" was supplemented with various lipids; triolein (trioleic acid glyceride) in a proportion of 5 % to obtain Fragrance Composition "C-3"; isostearic acid in a proportion of 3 % to give Fragrance Composition "C-4"; cholesterol in a proportion of 0.3 % to obtain Fragrance Composition "C-5"; cholesteryl isostearate (cholesterol ester) in a proportion of 0.2 % to obtain Fragrance Composition "C-6".

Portions of the above-mentioned "Downy care" were then supplemented with "C-1", "C-3", "C-4", "C-5" and "C-6" in a proportion of 0.5 %, respectively, so as to obtain products "Softener-2" to "Softener-6".

**Table 12**

| Fragrance Composition for Softener "C-1" | |
|---|---|
| Benzyl salicylate | 35 parts |
| Benzyl acetate | 40 |
| Galaxolide (IFF) | 50 |
| Geraniol | 10 |
| Methyl dihydrojasmonate | 15 |
| Heliotropine | 15 |
| α-hexylcinnamic aldehyde | 60 |
| Iso-E-super (IFF) | 10 |
| Lilial (GIVAUDAN ROURE S.A. | 60 |
| Linalool | 100 |
| Methyl ionone | 30 |
| Tonalide (PFW) | 25 |
| Vertenex (IFF) | 10 |
| Rose Takasago base (TAKASAGO) | 40 |

Cotton towels were washed with non-perfumed detergents, so as to remove any smelling element. Water was then removed from the towels. After 30 min, the towels were treated respectively with Softener-1 to Softener-6.

The cotton towels were dipped for 30 min in 300 ml of softener solution containing one g of softener. The towels were taken out from the softener solution, dehydrated, rinsed twice with 200 ml of water, and further dehydrated.

A group of ten panellists evaluated the intensity of scents emanating from the towels immediately after water-removal, and after 1, 2 and 24 hours.

### Evaluation rating

Panellists' evaluations on the intensity of remaining scent (enduring fragrance) were rated into 5 categories on the basis of the definitions mentioned for 4^{th} Embodiment.

The results are presented in Table 13.

**Table 13**

| Evaluation of remaining scents | | | | |
|---|---|---|---|---|
| | Immediately after water-removal | After 1 h | After 2 h | After 24 h |
| Softener-1 (10^{th} Embodiment) | 3.50 | 3.40 | 3.25 | 2.40 |
| Softener-2 (Com. Ex.9) | 3.50 | 2.80 | 2.50 | 1.60 |
| Softener-3 (Com. Ex.10) | 3.50 | 2.80 | 2.50 | 1.60 |
| Softener-4 (Com. Ex.11) | 3.50 | 2.80 | 2.50 | 1.60 |
| Softener-5 (Com. Ex.12) | 3.50 | 2.80 | 2.50 | 1.60 |
| Softener-6 (Com. Ex.13) | 3.50 | 2.80 | 2.50 | 1.60 |

As can be seen from the above table, Softener-1 (10^{th} Embodiment) containing the inventive lipid composition produce a significantly better scent retention effect, compared to Softener-2 (Comparative Example-9) containing no such lipid composition added. The same results have been obtained when compared to Softeners-3 to Softener-6.

### [11^{th} to 14^{th} Embodiments, Comparative Example 14]

### Preparation of softeners and evaluation on towels

In 11^{th} to 14^{th} Embodiments, the lipid compositions of Preparation Examples 1 to 3 were added to Fragrance Composition for Softener "D-1" (Table 14) in proportions defined below, to give 4 kinds of Fragrance Compositions "D-2" to "D-5":
(1) Fragrance Composition "D-2": lipid composition 1 of Preparation Example 1 was added to "D-1" in a proportion of 0.2 %;
(2) Fragrance Composition "D-3": lipid composition 2 of Preparation Example 2 was added to "D-1" in a proportion of 0.2 %;
(3) Fragrance Composition "D-4": lipid composition 3 of Preparation Example 3 was added to "D-1" in a proportion of 0.2 %;
(4) Fragrance Composition "D-5": lipid composition 3 of Preparation Example 3 is added to "D-1" in a proportion of 0.4 %.

In 11^{th} to 14^{th} Embodiments, a group of base softeners was prepared according to Formulation 4 in Table 15 below. To them were added respectively Fragrance Compositions "D-2" to "D-5" in a proportion of 1.0 %, so as to obtain products "Softener-7" to "Softener-10".

In Comparative Example 14, Fragrance Composition for Softener "D-1" was added to the base softener in a proportion of 1.0 %, so as to obtain a product "Softener-11".

**Table 14**

| Fragrance Composition for Softener "D-1" | |
|---|---|
| Isoamyl acetate | 1.5 parts |
| Limonene | 10.0 |
| Linalool | 10.0 |
| Tricyclodecenyl acetate ("Erika acetate" of TAKASAGO) | 5.0 |
| γ-methyl ionone | 3.0 |
| β-ionone | 3.0 |
| Lilial (GIVAUDAN ROURE S.A.) | 6.0 |
| Galaxolide (IFF) | 6.0 |
| Tonalide (PFW) | 3.5 |
| Hexylcinnamic aldehyde | 6.0 |
| Benzyl salicylate | 7.5 |
| Heliotropine | 1.5 |
| Methyl dihydrojasmonate ("Hedione" of FIRMENICH S.A.) | 1.5 |
| Rose Fragrance Base (TAKASAGO) | 20.0 |
| Dipropyleneglycol | 25.0 |

**Table 15**

| Formulation-4 for base softeners | |
|---|---|
| Ethyleneglycol | 6.0 parts |
| Polyoxyethylene oleylcetyl ether | 2.0 |
| Dibutylhydroxy toluene | 0.1 |
| Purified water | 71.1 |
| Anhydrous Sodium sulfate | 0.8 |
| Imidazoline-type cationic surfactant ("Cation SF-75PA" of SANYO KASEI KOGYO) | 20.0 |

Textiles to be tested (mixed fibres composed of cotton, chemical fibres, etc.) were wetted by washing with distilled water. One gram of each of Softener-7 to Softener-11 supra was dissolved respectively in 200 ml of water. The test textiles were dipped in respective 200 ml of softener solutions for one hour. The water was removed from the textiles, and the latter were dried in a room under the sun.

A group of ten panellists evaluated the intensity of scents emanating from the textiles after 24 and 72 hours of drying.

### Evaluation rating

Panellists' evaluations on the intensity of remaining scent (enduring fragrance) were rated into 5 categories on the basis of the definitions mentioned for 4^{th} Embodiment.

The results of the evaluations are presented in Table 16.

**Table 16**

| Evaluation of remaining scents | | |
|---|---|---|
| | After 24 h | After 72 h |
| Softener-7 (12^{th} Embodiment) | 3.50 | 2.50 |
| Softener-8 (13^{th} Embodiment) | 3.30 | 2.30 |
| Softener-9 (14^{th} Embodiment) | 2.40 | 1.50 |
| Softener-10 (15^{th} Embodiment) | 2.40 | 1.50 |
| Softener-11 (16^{th} Embodiment) | 1.00 | 0.50 |

As can be seen from the above table, Softeners-7 to 10 (11^{th} to 14^{th} Embodiments) containing the inventive lipid composition exhibit a significantly better scent retention effect, compared to Softener-11 (Comparative Example-14) containing no such lipid composition.

Fragrance Compositions for Softeners were prepared according to Preparation Examples 1, 2 and 3, by varying the types of inventive lipid compositions as described below.

Lipid compositions were prepared according to Preparation Examples 1 and 2, using a ceramide compound A₁ of the formula (I) and/or (IV) and a ceramide compound A₂ of the formula (II) and /or (V), and the softeners of the 11^{th} and 12^{th} Embodiments were then prepared using the above lipid compositions.

The softeners containing these lipid compositions produced a marked scent-retaining effect.

A lipid composition was prepared according to Preparation Example 3, using a pseudo-ceramide compound A₃ of the formula (III), and the softeners of the 13^{th} and 14^{th} Embodiments were then prepared using this lipid composition.

The softeners using a pseudo-ceramide (13^{th} and 14^{th} Embodiments) produced a slightly lesser scent-retaining effect than the softeners using a ceramide (11^{th} and 12^{th} Embodiments), but yielded nonetheless a better effect than the softener containing no lipid composition (Comparative Example 14).

In the 11^{th} to 14^{th} Embodiments and Comparative Example 14, the towels (fibres) were dried for 72 hours and subjected to analyses by "Headspace GC-MS".

In particular, the intensity of towels' smell was first evaluated. The towels (fibres) were then enclosed e.g. in a mayonnaise bottle having a volume of 300 ml, and their headspace was compared. To this end, a fibre assembly for Solid Phase Micro Extraction "SPME", manufactured by Supelco Inc. for gas chromatography use, was inserted into the bottle up to the level of the deposed fibres, and collected an air sample for 10 min. The air sample was then introduced into an apparatus "GC-MS" and analysed.

The area value of main fragrance ingredients, detected in the towels after 72 hours of drying, was calculated, giving the following results.

### Total amounts of fragrance ingredients detected by "Headspace GC-MS"

| | |
|---|---|
| Comparative Example 14 | : 362, 000, 000 |
| 11^{th} Embodiment | : 433, 000, 000 |
| 12^{th} Embodiment | : 432, 000, 000 |
| 13^{th} Embodiment | : 413, 000, 000 |
| 14 ^{th} Embodiment | : 425, 000, 000 |

As is obvious from the above, the towels washed with the softeners containing the lipid composition of the invention (11^{th} to 14^{th} Embodiments) contained a higher fragrance amount 72 hours after the use, detected by "Headspace GC-MS", as compared to the softener (Comparative Example 14) containing no such lipid composition.

In the above analyses, all the main fragrance ingredients (isoamyl acetate, methyl ionone, linalool, Erica acetate, ionone, galaxolide, tonalide and the like) were detected in a higher level in 11^{th} to 14^{th} Embodiments than in Comparative Example 14.

The endurance of the fragrances in the softeners containing the inventive lipid composition could thus be confirmed by both smelling tests and the physical analyses by "Headspace GC-MS".

### [15^{th} to 17^{th} Embodiments, Comparative Example 15]

### Preparation of after-wash softeners and evaluation on towels

In 15^{th} to 17^{th} Embodiments, the following 4 types of Fragrance Compositions for Softeners "D-1" and "D-6" to "D-8" were prepared using Fragrance Composition for Softener "D-1" of the 11^{th} to 14^{th} Embodiments:
(1) Fragrance Composition "D-1":as prepared in 11^{th} to 14^{th} Embodiments;
(2) Fragrance Composition "D-6": lipid composition 1 of Preparation Example 1 was added to "D-1" in a proportion of 0.25 %;
(3) Fragrance Composition "D-7": lipid composition 1 of Preparation Example 1 was added to "D-1" in a proportion of 0.5 %;
(4) Fragrance Composition "D-8": lipid composition 1 of Preparation Example 1 was added to "D-1" in a proportion of 1.0 %.

In 15^{th} to 17^{th} Embodiments, a group of base softeners was prepared according to Formulation 4 supra. To them were added respectively Fragrance Compositions "D-6" to "D-8" in a proportion of 0.5 %, so as to obtain products "Softener-12" to "Softener-14".

In Comparative Example 15, the base softener was prepared according to Formulation 4. To this was added Fragrance Composition for Softener "D-1" in a proportion of 0.5 %, so as to obtain a product "Softener-15".

Cotton towels to be tested were washed with a detergent containing no fragrance, so as to remove any perfume element. Water was then removed from the towels. The towels were allowed to stand for 30 min, and treated with Softener 12 to Softener 15 as follows.

One gram of each of Softener-12 to Softener-15 supra was respectively dissolved in 300 ml of water. The test towels were dipped in respective 300 ml of softener solutions for 30 min, and taken out therefrom. The water was removed from the towels. The latter were rinsed twice with 200 ml of water, and dehydrated.

A group of ten panellists evaluated the intensity of scents emanating from the towels, immediately after the water-removal, and respectively one, 2, 24 and 48 hours after drying.

### Evaluation rating

Panellists' evaluations on the intensity of remaining scent (enduring fragrance) were rated into 5 categories according to the definitions given for 4^{th} Embodiment.

The results of the evaluations are presented in Table 17.

**Table 17**

| Evaluation of remaining scents | | | | | |
|---|---|---|---|---|---|
| | Immediately after water-removal | After 1 h | After 2 h | After 24 h | After 48 h |
| Softener-12 (15^{th} Embod.) | 3.50 | 3.40 | 3.20 | 2.50 | 2.00 |
| Softener-13 (16^{th} Embod.) | 3.50 | 3.40 | 3.30 | 3.30 | 2.30 |
| Softener-14 (17^{th} Embod.) | 3.50 | 2.80 | 3.40 | 3.30 | 2.50 |
| Softener-15 (Com.Ex.l5) | 3.50 | 2.80 | 2.50 | 1.70 | 0.60 |

As can be seen from the above table, the towels treated with Softeners-12 to Softener 14 (15^{th} to 17^{th} Embodiments), to which lipid composition 1 of Preparation Example 1 are added in a varied quantity, exhibit a significantly better scent retention effect even after 48 hours, compared to the towels treated with Softener-15 (Comparative Example-15) containing no such lipid composition.

The softeners of the invention thus show a remarkable scent retention capacity, not only immediately after washing, but also after the drying.

In 15^{th} to 17^{th} Embodiments and Comparative Example 15, the towels (fibres) were dried for 48 hours and subjected to analyses by "Headspace GC-MS".

In particular, the intensity of towels' smell was first evaluated. The towels (fibres) were then enclosed e.g. in a mayonnaise bottle having a volume of 300 ml, and their headspace was compared. To this end, a fibre assembly for Solid Phase Micro Extraction "SPME", manufactured by Supelco Inc. for gas chromatography use, was inserted into the bottle up to the level of the deposed fibres, and collected an air sample for 10 min. The air sample was then introduced into an apparatus "GC-MS" and analysed.

The area value of main fragrance ingredients, detected in the towels after 48 hours of drying, was calculated, giving the following results.

### Total amounts of fragrance ingredients detected by "Headspace GC-MS"

| | |
|---|---|
| Comparative Example 15 | : 45, 421, 993 |
| 15^{th} Embodiment | : 72, 214, 925 |
| 16^{th} Embodiment | : 93, 437, 258 |
| 17^{th} Embodiment | : 128, 413, 220 |

As is obvious from the above, the towels washed with the softeners containing the lipid composition of the invention (15^{th} to 17^{th} Embodiments) contained a higher fragrance amount 48 hours after the use, detected by "Headspace GC-MS", as compared to the softener (Comparative Example 15) containing no such lipid composition.

In the above analyses, all the main fragrance ingredients (benzyl acetate, citronellol, lilial, Iso-E-super, hexyl salicylate, hexyl cinnamic aldehyde and the like) were detected in a higher level in 15^{th} to 17^{th} Embodiments than in Comparative Example 15.

The endurance of the fragrances in the softeners containing the inventive lipid composition could thus be confirmed by both smelling tests and the physical analyses by "Headspace GC-MS".

### [18^{th} to 20^{th} Embodiments, Comparative Example 19]

### Preparation of hair conditioners

The lipid compositions of Preparation Example 1 were added to Fragrance Composition for Conditioners "E-1" (Table 18) in various proportions to obtain Fragrance Compositions "E-2 to "E-4" as follows.
(1) Fragrance Composition "E-2": lipid composition 1 was added to Fragrance Composition "E-1" in a proportion of 0.2 %;
(2) Fragrance Composition "E-3": lipid composition 1 was added to Fragrance Composition "E-1" in a proportion of 0.5 %;
(3) Fragrance Composition "E-4": lipid composition 1 was added to Fragrance Composition "E-1" in a proportion of 1.0 %.

In 18^{th} to 20^{th} Embodiments, a group of base conditioners were prepared according to Formulation 5 in Table 19 below. To these were added Fragrance Compositions for Conditioners "E-2" to "E-4" in a proportion of 0.5 % respectively, so as to obtain products "Conditioner-1" to "Conditioner-3".

In Comparative Example 19, a base conditioner was prepared according to Formulation 5. To this was added Fragrance Composition "E-1" (Table 18) in a proportion of 0.5 %, to obtain a product "Conditioner-4".

**Table 18**

| Fragrance Composition for Conditioners "E-1" | |
|---|---|
| Orange terpene | 10.0 parts |
| Linalool | 10.0 |
| Tricyclodecenyl acetate ("Erica acetate" de TAKASAGO) | 5.0 |
| γ-methyl ionone | 3.0 |
| β-ionone | 3.0 |
| Lilial (GIVAUDAN ROURE S.A.) | 7.5 |
| Galaxolide (IFF) | 10.0 |
| Tonalide (PFW) | 3.0 |
| Hexylcinnamic aldehyde | 4.0 |
| Hexyl salicylate | 4.5 |
| Benzyl salicylate | 5.5 |
| Heliotropine | 1.5 |
| Methyl dihydrojasmonate ("Hedione" of FIRMENICH S.A.) | 14.5 |
| Rose Fragrance Base (TAKASAGO) | 10.0 |
| Dipropylene glycol | 5.0 |
| Ethylene brassilate ("Musk T" of TAKASAGO) | 3.0 |
| Hydroxycitronellal ("Laurinal" de TAKASAGO) | 5.0 |

**Table 19**

| Formulation 5 for base conditioners | |
|---|---|
| Purified water | 42.03 parts |
| Ortho-[2-hydroxy-3-(trimethylammonio)propyl]hydroxyethyl cellulose chloride | 0.200 |
| Sodium hydroxide | 0.005 |
| Methyl p-oxybenzoate | 0.20 |
| Propyl p-oxybenzoate | 0.10 |
| Tetrasodium edetate | 0.05 |
| Polyoxyethylene cetylether | 0.500 |
| Cetanol | 2.000 |
| Behenyl alcohol | 2.000 |
| Stearyltrimethylammonium chloride | 3.000 |
| Distearyldimethylammonium chloride | 0.200 |
| Cetyl 2-ethylhexanoate | 0.500 |
| Methyl polysiloxane | 2.000 |

Non-perfumed shampoos were prepared according to Formulation 1 of the 4^{th} Embodiment. Bundles of homogenous human hairs (20 g) were dipped in 50 ml of warm water at 40°C and soaked for 20 min. The bundles of hairs were respectively washed with one g of the above non-perfumed shampoo, and rinsed with 100 ml of warm water, so as to remove any smelling element.

The water portion was then removed from the bundles of hairs, and the latter were allowed to stand for 10 min. The bundles of hairs were then treated with Conditioner-1 to Conditioner-4 as follows.

One gram of each of Conditioner-1 to 4 supra was respectively dissolved in 50 ml of warm water, so as to prepare corresponding conditioner solutions. The bundles of hairs were respectively dipped in 50 ml of conditioner solutions for 5 min. They were taken out from the solutions, dehydrated, rinsed with 100 ml of warm water, and further dehydrated..

A group of ten panellists evaluated the intensity of scents emanating from the bundles of hairs, immediately after the final dehydration, and respectively one, 2, 24 and 48 hours after drying.

### Evaluation rating

Panellists' evaluations on the intensity of remaining scent (enduring fragrance) were rated into 5 categories according to the definitions given for 4^{th} Embodiment.

**Table 20**

| Evaluation of remaining scents | | | | | |
|---|---|---|---|---|---|
| | Immediately after final dehydration | After | After | After | After |
| | | 1 h | 2 h | 24 h | 48 h |
| Conditioner-1 (18^{th} Embod.) | 3.50 | 3.40 | 3.20 | 2.50 | 2.30 |
| Conditioner-2 (19^{th} Embod.) | 3.50 | 3.40 | 3.20 | 2.40 | 2.00 |
| Conditioner-3 (20^{th} Embod.) | 3.50 | 3.40 | 3.20 | 2.50 | 2.30 |
| Conditioner-4 (Com. Ex.19) | 3.50 | 3.40 | 3.20 | 1.60 | 0.90 |

As can be seen from the above table, Conditioner-1 to 3 of the invention (which contain the inventive lipid compositions) exhibited a significantly stronger scent retention effect, compared to Conditioner-4 (which contains no such lipid composition).

### [21^{st} Embodiment, Comparative Example 20]

### Preparation of creams

The lipid composition 1 of Preparation Example 1 was added to Fragrance Composition for Creams "F-1" (Table 21) in a proportion of 0.4 %, to obtain Fragrance Composition "F-2".

In 21^{st} Embodiment, 20 g of base cream were prepared according to Formulation 6 in Table 22 below. To this was added Fragrance Composition "F-2" in a proportion of 0.2 % to obtain a product "Cream-2".

In Comparative Example 20, 20 g of base cream were prepared according to Formulation 6. To this was added Fragrance Composition "F-1" in a proportion of 0.2 g, to obtain a product "Cream-1".

The obtained creams were kept in the dark for about 3 months, and subjected to the evaluations of remaining scent and the measures of scent intensity.

**Table 21**

| Fragrance Composition for Creams "F-1" | |
|---|---|
| Hydroxycitronellal ("Laurinal" of TAKASAGO) | 2.0 parts |
| Linalool | 2.0 |
| Linalyl acetate | 3.0 |
| Nerolidol | 2.0 |
| Rose Fragrance Base (TAKASAGO) | 15.0 |
| Isocamphyl cyclohexanol ("Santalex" of TAKASAGO) | 3.0 |
| Tonalide (PFW) | 1.0 |
| Preparation Base (TAKASAGO) | 5.5 |
| Benzyl salicylate | 7.0 |
| Citronellol | 1.0 |
| Galaxolide (IFF) | 4.0 |
| Methyl dihydrojasmonate ("Hedione" of FIRMENICH S.A.) | 5.0 |
| Helional (IFF) | 14.5 |
| Dihydromyrcenol | 3.0 |
| Dipropylene glycol | 20.0 |
| Farnesol | 1.5 |
| Cis-3-hexenyl salicylate | 4.5 |
| Lilial (GIVAUDAN ROURE S.A.) | 5.5 |

**Table 22**

| Formulation 6 for Creams | |
|---|---|
| Fragrance | 1.0 parts |
| Bleached beeswax | 1.0 |
| 1,3-butanediol | 5.0 |
| Cetyl octanoate | 1.5 |
| Squalane | 30.0 |
| Cetanol | 4.0 |
| Monostearic acid polyethyleneglycol | 1.4 |
| Glyceryl monostearate | 2.4 |
| Paraben | 0.2 |

A given amount of Cream-1 (Comparative Example 20) was painted on the back of the left hand, while the same amount of Cream 2 (21^{st} Embodiment) was painted on the back of the right hand.

A group of 20 panellists evaluated the remaining scents respectively 3 and 5 hours after the painting. The results are presented in Table 23.

**Table 23**

| The remaining scents are: | | |
|---|---|---|
| | stronger in Cream-1 | stronger in Cream-2 |
| | (Number of panellists) | (Number of panellists) |
| Immediately after the painting | 10 | 10 |
| After 3 h | 5 | 15 |
| After 5 h | 2 | 18 |

As can be seen from the above table, the intensities of remaining scents could not be differentiated between Cream-1 and Cream-2, when compared immediately after the painting. However, when compared after 3 and 5 hours, Cream-2 of the invention produced a much stronger remaining scent.

### [22^{nd} and 23^{rd} Embodiments, Comparative Examples 21 and 22]

### Preparation of fine fragrances

The lipid composition of Preparation Example 1 was added to Fragrance Composition "G-1" in Table 24 below (similar to commercial perfumed water) in a proportion of 1.0 %, so as to obtain Fragrance Composition "G-2".

In 22^{nd} Embodiment, Fragrance Composition "G-2" was diluted 5 times with ethanol to obtain a product "Perfumed Water-2".

In Comparative Example 21, Fragrance Composition "G-1" was diluted 5 times with ethanol to obtain a product "Perfumed Water-1".

Further, in 23^{rd} Embodiment, Fragrance Composition "G-2" was diluted 20 times with ethanol to obtain a product "Eau de Cologne-2".

Likewise, in Comparative Example 22, Fragrance Composition "G-1" was diluted 20 times with ethanol to obtain a product "Eau de Cologne-1".

The above perfumed water and Eau de Cologne were subjected to the evaluations of remaining scents and the measures of scent intensities.

**Table 24**

| Fragrance Composition "G-1" | |
|---|---|
| Benzyl salicylate | 7.0 parts |
| Citronellol | 1.3 |
| Dihydromyrcenol | 2.7 |
| Dipropyleneglycol | 20.0 |
| Farnesol | 1.5 |
| Galaxolide (IFF) | 4.0 |
| Methyl dihydrojasmonate ("Hedione" of FIRMENICH S.A.) | 5.0 |
| Helional (IFF) | 14.5 |
| Cis-3-hexenyl salicylate | 4.5 |
| Lilial (GIVAUDAN ROURE S.A.) | 5.5 |
| Hydroxycitronellal ("Laurinal" of TAKASAGO) | 2.5 |
| Linalool | 2.0 |
| Linalyl acetate | 3.0 |
| Nerolidol | 2.0 |
| Rose Fragrance Base (TAKASAGO) | 20.0 |
| Isocamphyl cyclohexanol ("Santalex" of TAKASAGO) | 3.0 |
| Tonalide (PFW) | 1.0 |
| Preparation Base (Takasago) | 0.5 |

A given amount of Perfumed Water-1 (Comparative Example 21) was painted on the back of the left hand, while the same amount of Perfumed Water-2 (22^{nd} Embodiment) was painted on the back of the right hand.

A group of 20 panellists evaluated the remaining scents immediately after the painting, and after 2 and 4 hours. The results are presented in Table 25.

**Table 25**

| Perfumed Water | | | |
|---|---|---|---|
| (digits: number of panellists who replied) | | | |
| The intensity of remaining scents is: | Immediately after | After 2 h | After 4 h |
| greater in Perfumed Water-1 (Com. Ex. 21) | 2 | 3 | 0 |
| the same for Perfumed Water-1 & -2 | 18 | 12 | 2 |
| greater in Perfumed Water-2 (22^{nd} Embod.) | 0 | 5 | 18 |

As can be seen from the above table, the intensities of remaining scents could not be differentiated between Perfumed Water-1 and Perfumed Water-2, when compared immediately after the painting. However, when compared after 4 hours, Perfumed Water-2 of the invention produced a much stronger remaining scent.

A given amount of Eau de Cologne-1 (Comparative Example 22) was painted on the back of the left hand, while the same amount of Eau de Cologne-2 (23^{rd} Embodiment) was painted on the back of the right hand.

A group of 20 panellists evaluated the remaining scents immediately after the painting, and after 2 and 4 hours. The results are presented in Table 26.

**Table 26**

| Eau de Cologne | | | |
|---|---|---|---|
| (digits: number of panellists who replied) | | | |
| The intensity of remaining scents is: | Immediately after | After 2 h | After 4 h |
| greater in Eau de Cologne-1 (Com. Ex. 22) | 2 | 4 | 0 |
| the same for Eaux de Cologne-1 and -2 | 18 | 12 | 2 |
| greater in Eau de Cologne-2 (23^{rd} Embod.) | 0 | 4 | 18 |

As can be seen from Table 26 above, the intensities of remaining scents could not be differentiated between Eau de Cologne-1 and Eau de Cologne-2, when compared immediately after the painting. However, when compared after 4 hours, Eau de Cologne-2 of the invention produced a much stronger remaining scent.

As shown in the foregoing embodiments, when the inventive lipid compositions containing a ceramide and/or a pseudo-ceramide are added to a fragrance composition, the fragrances in the latter composition acquire a long-lasting nature. Further, when such fragrance compositions are added to different products such as cosmetics, hair care products, body cleansers, skin care products and fabric care products, the fragrances in the latter products are imparted with enduring capacity. Furthermore, these enduring fragrances are retained remarkably well in the hairs and articles after washing, or on human skin.

The fragrance or cosmetic compositions of the invention can thus be applied to various cosmetics and toiletries.

## Claims

1. Use of a composition comprising a sphingolipid-based composition (A), and an ingredient (B) comprising at least one compound chosen from the group consisting of sterol-based compounds, for reinforcing the endurance of scents in a fragrance compound or composition.

2. Use according to claim 1, wherein said sphingolipid-based composition (A) comprises:
a) at least a first ceramide ingredient (A₁) selected from the group consisting of 2-acylaminoalkan-1,3-diol and optically active forms thereof represented by the formula I: 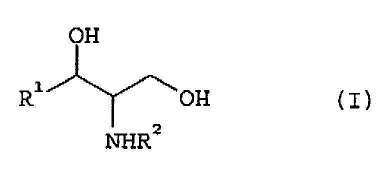 in which R¹ signifies a straight-chain alkyl group having 9 to 17 carbon atoms, and R² signifies a straight-chain acyl group having 14 to 24 carbon atoms; and
b) at least a second ceramide ingredient (A₂) selected from the group consisting of 2-acylaminoalkan-1,3-diol and optically active forms thereof represented by the formula II: 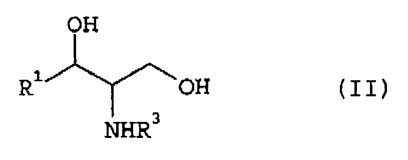
in which R¹ signifies a straight-chain alkyl group having 9 to 17 carbon atoms, and R³ signifies an acetyl group or a straight-chain acyl group having 2 to 24 carbon atoms with a hydroxy group at α- or β-position.

3. Use according to claim 1 or 2, wherein said fragrance-retaining composition further comprises at least one ingredient (C) selected from the group consisting of cholesterol esters and higher fatty acids.

4. Use according to any one of claims 1 to 3, wherein said first ceramide ingredient (A₁) comprises a (2S,3R)-2-acylaminoalkan-1,3-diol represented by the formula (IV): 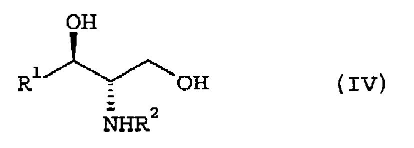 in which R¹ and R² are as defined for the formula (I).

5. Use according to any one of claims 1 to 4, wherein said second ceramide ingredient (A₂) comprises a (2S,3R)-2-acylaminoalkan-1,3-diol represented by the formula (V): 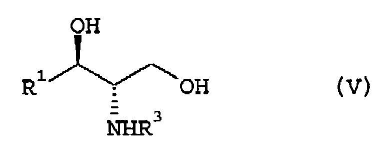 in which R¹ and R³ are as defined for the formula (II).

6. Use according to claim 1, wherein said sphingolipid-based composition (A) comprises:
a) at least one pseudo-ceramide ingredient (A₃) selected from the group consisting of amide derivatives represented by the formula III: 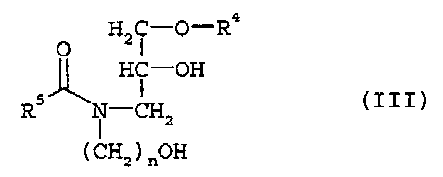 in which R⁴ signifies a straight-chain or branched, saturated or non-saturated hydrocarbon group having 10 to 26 carbon atoms; R⁵ signifies a straight-chain or branched, saturated or non-saturated hydrocarbon group having 9 to 25 carbon atoms; and n signifies a natural number ranging from 2 to 6; and
b) at least one ingredient (C) selected from the group consisting of cholesterol esters and higher fatty acids.

7. Use according to any one of claims 1 to 6, wherein said fragrance-retaining composition has a liquid-crystal structure.

## Patentansprüche

1. Verwendung einer Zusammensetzung, umfassend eine Zusammensetzung auf Sphingolipidbasis (A) und einen Inhaltsstoff (B), umfassend mindestens eine Verbindung, ausgewählt aus der Gruppe, bestehend aus Verbindungen auf Sterolbasis, zum Verstärken der Dauerhaftigkeit von Düften in einer Duftstoffverbindung oder -zusammensetzung.

2. Verwendung nach Anspruch 1, wobei die Zusammensetzung auf Sphingolipidbasis (A)
a) mindestens einen ersten Ceramidinhaltsstoff (A₁), ausgewählt aus der Gruppe, bestehend aus 1-Acylaminoalkan-1,3-diol und optisch aktiven Formen davon, dargestellt durch die Formel I 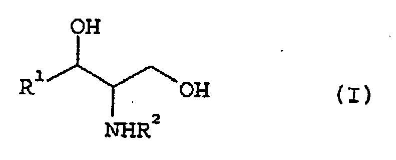 in welcher R¹ eine geradkettige Alkylgruppe mit 9 bis 17 Kohlenstoffatomen darstellt und R² eine geradkettige Acylgruppe mit 14 bis 24 Kohlenstoffatomen darstellt, und
b) mindestens einen zweiten Ceramidinhaltsstoff (A₂), ausgewählt aus der Gruppe, bestehend aus 2-Acylaminoalkan-1,3-diol und optisch aktiven Formen davon, dargestellt durch die Formel II 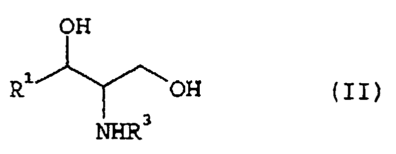 in welcher R¹ eine geradkettige Alkylgruppe mit 9 bis 17 Kohlenstoffatomen darstellt und R³ eine Acetylgruppe oder eine geradkettige Acylgruppe mit 2 bis 24 Kohlenstoffatomen mit einer Hydroxylgruppe an α- oder β-Position darstellt,
umfasst.

3. Verwendung nach Anspruch 1 oder 2, wobei die Duft-erhaltende Zusammensetzung ferner mindestens einen Inhaltsstoff (C) umfasst, ausgewählt aus der Gruppe, bestehend aus Cholesterolestern und höheren Fettsäuren.

4. Verwendung nach einem der Ansprüche 1 bis 3, wobei der erste Ceramidinhaltsstoff (A₁) ein (2S,3R)-2-Acylaminoalkan-1,3-diol, dargestellt durch die Formel (IV) 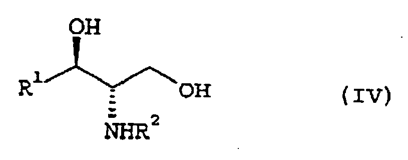 in welcher R¹ und R² wie für die Formel (I) definiert sind, umfasst.

5. Verwendung nach einem der Ansprüche 1 bis 4, wobei der zweite Ceramidinhaltsstoff (A₂) ein (2S,3R)-2-Acylaminoalkan-1,3-diol umfasst, dargestellt durch die Formel (V) 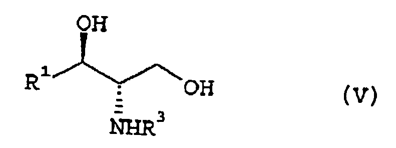 in welcher R¹ und R³ wie für die Formel (II) definiert sind.

6. Verwendung nach Anspruch 1, wobei die Zusammensetzung auf Sphingolipidbasis (A)
a) mindestens einen pseudo-Ceramidinhaltsstoff (A₃), ausgewählt aus der Gruppe, bestehend aus Amidderivaten, dargestellt durch die Formel III 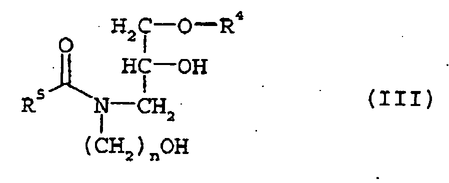 in welcher R⁴ eine geradkettige oder verzweigtkettige, gesättigte oder ungesättigte Kohlenwasserstoffgruppe mit 10 bis 26 Kohlenstoffatomen darstellt, R⁵ eine geradkettige oder verzweigtkettige, gesättigte oder ungesättigte Kohlenwasserstoffgruppe mit 9 bis 25 Kohlenstoffatomen darstellt, und n eine natürliche Zahl im Bereich von 2 bis 6 darstellt, und
b) mindestens einen Inhaltsstoff (C), ausgewählt aus der Gruppe, bestehend aus Cholesterolestern und höheren Fettsäuren,
umfasst.

7. Verwendung nach einem der Ansprüche 1 bis 6, wobei die Duft-erhaltende Zusammensetzung eine Flüssigkristallstruktur aufweist.

## Revendications

1. Utilisation d'une composition comprenant une composition (A) à base de sphingolipide et un ingrédient (B) comprenant au moins un composé choisi dans l'ensemble constitué de composés à base de stérol pour renforcer la durée des senteurs dans un composé parfumant ou une composition parfumante.

2. Utilisation selon la revendication 1, dans laquelle ladite composition (A) à base de sphingolipide comprend :
a) au moins un premier ingrédient à base de céramide (A₁) choisi dans l'ensemble constitué de 2-acylaminoalcane-1,3-diol et des formes optiquement actives de celui-ci, représenté par la formule I : 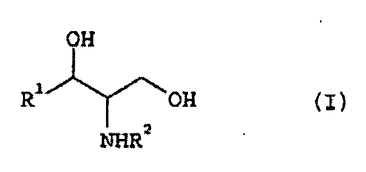 dans laquelle R¹ signifie un groupe alkyle à chaîne linéaire ayant de 9 à 17 atomes de carbone et R² signifie un groupe acyle à chaîne linéaire ayant de 14 à 24 atomes de carbone ; et
b) au moins un second ingrédient à base de céramide (A₂) choisi dans l'ensemble constitué de 2-acylaminoalcane-1,3-diol et des formes optiquement actives de celui-ci, représenté par la formule II : 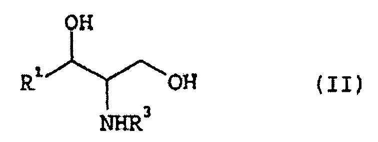 dans laquelle R¹ signifie un groupe alkyle à chaîne linéaire ayant de 9 à 17 atomes de carbone et R³ signifie un groupe acétyle ou un groupe acyle à chaîne linéaire ayant de 2 à 24 atomes de carbone avec un groupe hydroxy en position α ou β.

3. Utilisation selon la revendication 1 ou 2, dans laquelle ladite composition retenant un parfum comprend en outre au moins un ingrédient (C) choisi dans l'ensemble constitué d'esters de cholestérol et d'acides gras supérieurs.

4. Utilisation selon l'une quelconque des revendications 1 à 3, dans laquelle ledit premier ingrédient à base de céramide (A₁) comprend un (2S,3R)-2-acylaminoalcane-1,3-diol représenté par la formule (IV) : 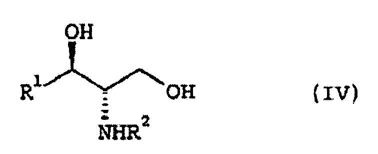 dans laquelle R¹ et R² sont tels que définis pour la formule (I).

5. Utilisation selon l'une quelconque des revendications 1 à 4, dans laquelle ledit second ingrédient à base de céramide (A₂) comprend un (2S,3R)-2-acylaminoalcane-1,3-diol représenté par la formule (V) : 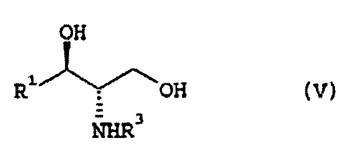 dans laquelle R¹ et R³ sont tels que définis pour la formule (II).

6. Utilisation selon la revendication 1, dans laquelle ladite composition (A) à base de sphingolipide comprend :
a) au moins un ingrédient à base de pseudo-céramide (A₃) choisi dans l'ensemble constitué de dérivés d'amides représentés par la formule III : 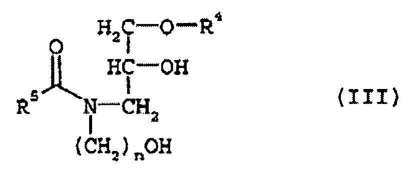 dans laquelle R⁴ signifie un groupe hydrocarboné à chaîne linéaire ou ramifiée, saturé ou non saturé, ayant de 10 à 26 atomes de carbone ; R⁵ signifie un groupe hydrocarboné à chaîne linéaire ou ramifiée, saturé ou non saturé, ayant de 9 à 25 atomes de carbone ; et n signifie un entier naturel compris entre 2 et 6 ; et
b) au moins un ingrédient (C) choisi dans l'ensemble constitué d'esters de cholestérol et d'acides gras supérieurs.

7. Utilisation selon l'une quelconque des revendications 1 à 6, dans laquelle ladite composition retenant un parfum a une structure à cristaux liquides.
